# EUROPEAN PATENT SPECIFICATION

(11) **EP 2 703 410 B1**
(45) Date of publication and mention of the grant of the patent: **14.10.2015**
(21) Application number: 12006113.0
(22) Date of filing: 28.08.2012
(51) Int. Cl.: C07K 7/64, C12P 21/00, A61K 38/12, A61P 31/10, A61P 35/00, C07K 7/56

(54) **Jagaricin, derivatives, and uses thereof**
Jagaricin, Derivate und ihre Verwendung
Jagaricine, dérivés et leurs utilisations

(43) Date of publication of application: 05.03.2014
(73) Proprietor: Leibniz-Institut für Naturstoff-Forschung und Infektionsbiologie, 07745 Jena (DE)
(72) Inventor: Bretschneider, Tom, 07747 Jena (DE); Graupner, Katharina, 07745 Jena (DE); Hertweck, Christian, 04105 Leipzig (DE); Lackner, Gerald, 07743 Jena (DE); Scherlach, Kirstin, 04105 Leipzig (DE)
(74) Representative: Forstmeyer, Dietmar

(56) References cited:
- EP-A1- 1 698 638
- US-A- 4 137 224

## Description

### Field of the invention

The present invention concerns a novel compound termed jagaricin, jagaricin derivatives, pharmaceutical compositions comprising these compounds, a method for producing jagaricin, and the the novel compound for use as fungicide or antitumor agent.

### Background of the invention

Microbial natural products are one of the most promising sources for novel drugs. This is, because natural products own an element of structural complexity which allows for the specific and effective inhibition of many protein targets. For instance, nonribosomally synthesized peptides (NRPs) or polyketides, are a prosperous source for new bioactive compounds (see, e.g. A. L. Harvey, Drug Discov. Today 2008, 13, 894; D. J. Newman, G. M. Cragg, J. Nat. Prod. 2012, 75, 311). Nonribosomal peptide synthetases (NRPSs) consist of different building blocks, so called modules, that are responsible for the activation and incorporation of one amino acid into the growing peptide chain at a time (R. Finking, M. A. Marahiel, Annu. Rev. Microbiol. 2004, 58, 453; D. Schwarzer, R. Finking, M. A. Marahiel, Nat. Prod. Rep. 2003, 20, 275). Thereby, every module can be further dissected into domains which exhibit one enzymatic function each. The adenylation-(A)-domain recognizes and activates the substrate, usually amino acids. These activated amino acids are transferred to the thiolation-(T)-domain (also peptidyl carrier protein-(PCP)-domain) that is responsible for the transport of the substrate between the other catalytic domains. The peptide bond formation is catalyzed by the condensation-(C)-domain. In addition to these core domain several modification domains, like epimerization-(E)-domains, can be a part of NRPSs (C. T. Walsh et al., Curr. Opin. Chem. Biol. 2001, 5, 525). The last module harbors a thioesterase-(Te)-domain that releases the peptide chain either as a linear or as a cyclic product (Finking and Marahiel, *loc. cit.*; Schwarzer, Finking, and Marahiel, *loc. cit.*).

The research on antifungal medication has been neglected in the past, since fungal diseases were considered as easily curable (R. Di Santo, Nat. Prod. Rep. 2010, 27, 1084; M. F. Vicente et al., Clin. Microbiol. Infect. 2003, 9, 15). However, an increasing need for antifungal drugs has emerged, as the incidents of severe fungal infections are continuously rising. Such fungal infections can be particularly dangerous for immunocompromized patients or persons who received invasive surgeries, especially in view of the fact that resistance against commonly used drugs arises among fungal human pathogens (R. Di Santo, *loc. cit.*; N. H. Georgopapadakou, T. J. Walsh, Science 1994, 264, 371).

Although much progress has been made in the development of antitumor agents, cancer is one of the leading causes of death. The most effective chemotherapeutics either interfere with the tumor cell cycle and division or bind to DNA and cause apoptosis through various downstream processes.

The motile Gram-negative bacterium *Janthinobacterium agaricidamnosum* causes the soft rot disease of mushrooms (S. P. Lincoln, T. R. Fermor, B. J. Tindall, Int. J. Syst. Bacteriol. 1999, 49 Pt 4, 1577). For *J. lividum*, a better investigated bacterium from the genus *Janthinobacterium,* secondary metabolite production has already been described (J. H. Johnson, A. A. Tymiak, Bolgar, M. S., J. Antibiot. 1990, 43, 920; J. O'Sullivan et al., J. Antibiot. 1990, 43, 913; A. Shirata et al., J. Sericult.Sci. Jpn. 1997, 66, 377). Accordingly, *Janthinobacterium agaricidamnosum* may also be a promising source for novel bioactive natural products.

Thus, a need remains to provide novel compounds or compositions that may be used to effectively treat fungal infections/diseases and/or cancer.

It is, therefore, an aim of the present invention to provide a novel compound, and derivatives thereof, with antifungal and/or antitumor activity; a pharmaceutical composition comprising the novel compound or derivatives thereof; the novel compound for use as fungicide or antitumor agent, and the novel compound for use in a method of preventing or treating a fungal disease or cancer. Preferably, such treatment is more effective and not as burdensome as current treatments and improves the lives of the patients.

### Summary and Description of the Invention

The present invention was made in view of the prior art and the needs described above, and, therefore, the object of the present invention is to provide a novel compound and derivatives thereof. In particular, jagaricin a novel secondary metabolite from the mushroom pathogen *Janthinobacterium agaricidamnosum* and derivatives thereof are provided. Another object of the invention is to provide pharmaceutical compositions comprising the novel compound or derivatives thereof. Other objects of the present invention are to provide a method for producing jagaricin, and the novel compound for use as fungicide or antitumor agent.

These objects are solved by the subject matter of the attached claims.

These and other aspects of the present invention will become apparent upon reference to the following detailed description and definitions.

The inventors established that a gene cluster coding for a nine modular NRPS is responsible for the biosynthesis of the novel secondary metabolite compound - jagaricin - in *Janthinobacterium agaricidamnosum.*

The inventors showed that the novel compound has strong antifungal activity against the major human pathogens *Candida albicans, Aspergillus fumigatus* and *Aspergillus terreus.*

The inventors also showed that the novel compound exhibits antiproliferative and cytotoxic activity.

The inventors further established that the novel compound has little or no antibacterial activity.

The inventors also established that the novel compound is involved in the soft rot infection process, but is not essential for pathogenicity.

Taken together, the inventors demonstrate that the novel cyclic lipopeptide jagaricin is produced by the mushroom pathogen *Janthinobacterium agaricidamnosum* (*J. agaricidamnosum*) and displays strong antifungal activities against the major human pathogenic fungi *C. albicans* and *Aspergillus* spp as well as antiproliferative activity against human umbilical vein endothelial cells HUVEC, human chronic myeloid leukemia cells K-562 and cytotoxic activity against human cervix carcinoma cells HeLa.

These results for the first time provide the secondary metabolite jagaricin and derivatives thereof, and allow a therapeutic, preventive and/or curative role to be conceived for it or a derivative thereof in the treatment of a fungal infection/disease and/or cancer. Accordingly, the present invention is directed to a compound of the general formula (I): 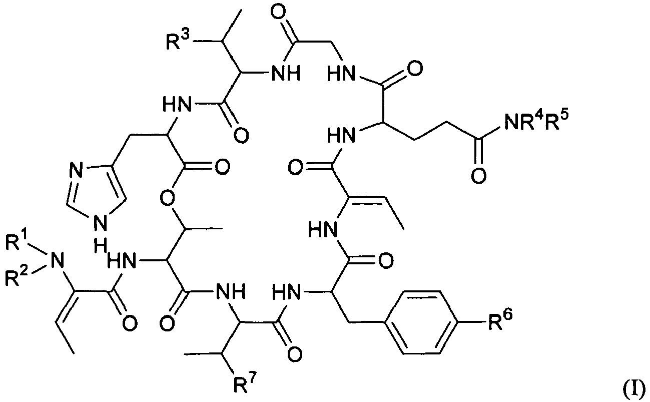 or a pharmacologically acceptable salt thereof, wherein
R¹ and R² can each independently represent a hydrogen atom, an optionally substituted alkyl group, an optionally substituted alkenyl group, or an optionally substituted alkinyl group, wherein one carbon atom in said alkyl, alkenyl, or alkynyl group may be replaced by an oxygen atom, a sulfur atom, C=O, NR¹⁰, CONR¹¹, or NR¹²CO at any chemically allowable position;
R¹⁰, R¹¹, and R¹² can each independently represent a hydrogen atom or an alkyl group having 1 to 6 carbon atoms;
R³ can be a hydrogen atom, a halogen atom, a hydroxyl group, a cyano group, a nitro group, an amino group, a mercapto group, an optionally substituted alkyl group having 1 to 6 carbon atoms, an alkoxy group having 1 to 6 carbon atoms, a SOCH₃ group, a SO₂CH₃ group, or an acyl group having 2 to 6 carbon atoms, or a polyethylene glycol group of formula -A-[CH₂-CH₂-O]ₙ-R²⁰, wherein A is -0-, -C(=O)-, -OC(=O)-, or -OC(=O)- (CH₂)ₘ-O-; m is an integer from 1 to 20; n is an integer of from 2 to 100, and R²⁰ is a hydrogen atom, a methyl group, or an ethyl group;
R⁴ and R⁵ can each independently represent a hydrogen atom, an optionally substituted alkyl group, an optionally substituted alkenyl group, or an optionally substituted alkinyl group, wherein one carbon atom in said alkyl, alkenyl, or alkynyl group may be replaced by an oxygen atom, a sulfur atom, C=O, NR¹³, CONR¹⁴, or NR¹⁵CO at any chemically allowable position;
R¹³, R¹⁴, and R¹⁵ can each independently represent a hydrogen atom or an alkyl group having 1 to 6 carbon atoms;
R⁶ can be a hydrogen atom, a halogen atom, a hydroxyl group, a cyano group, a nitro group, an amino group, a mercapto group, an optionally substituted alkyl group having 1 to 6 carbon atoms, an alkoxy group having 1 to 6 carbon atoms, a SOCH₃ group, a SO₂CH₃ group, an acyl group having 2 to 6 carbon atoms, or a polyethylene glycol group of formula -A-[CH₂-CH₂-O]ₙ-R²⁰, wherein A is -0-, -C(=O)-, -OC(=O)-, or -OC(=O)- (CH₂)ₘ-O-; m is an integer from 1 to 20; n is an integer of from 2 to 100, and R²⁰ is a hydrogen atom, a methyl group, or an ethyl group; and
R⁷ can be a hydrogen atom, a halogen atom, a hydroxyl group, a cyano group, a nitro group, an amino group, a mercapto group, an optionally substituted alkyl group having 1 to 6 carbon atoms, an alkoxy group having 1 to 6 carbon atoms, a SOCH₃ group, a SO₂CH₃ group, or an acyl group having 2 to 6 carbon atoms, or a polyethylene glycol group of formula -A-[CH₂-CH₂-O]ₙ-R²⁰, wherein A is -0-, -C(=O)-, -OC(=O)-, or -OC(=O)- (CH₂)ₘ-O-; m is an integer from 1 to 20; n is an integer of from 2 to 100, and R²⁰ is a hydrogen atom, a methyl group, or an ethyl group.

Compounds are generally described herein using standard nomenclature. For compounds having asymmetric centers, it should be understood that, unless otherwise specified, all of the optical isomers and mixtures thereof are encompassed. Compounds with two or more asymmetric elements can also be present as mixtures of diastereomers. In addition, compounds with carbon-carbon double bonds may occur in Z- and E- forms, with all isomeric forms of the compounds being included in the present invention unless otherwise specified. Where a compound exists in various tautomeric forms, a recited compound is not limited to any one specific tautomer, but rather is intended to encompass all tautomeric forms. Recited compounds are further intended to encompass compounds in which one or more atoms are replaced with an isotope, *i.e.*, an atom having the same atomic number but a different mass number. By way of general example, and without limitation, isotopes of hydrogen include tritium and deuterium and isotopes of carbon include ¹¹C, ¹³C, and ¹⁴C.

Compounds according to the formulas provided herein, which have one or more stereogenic center(s), have an enantiomeric excess of at least 50%. For example, such compounds may have an enantiomeric excess of at least 60%, 70%, 80%, 85%, 90%, 95%, or 98%. Some embodiments of the compounds have an enantiomeric excess of at least 99%. It will be apparent that single enantiomers (optically active forms) can be obtained by asymmetric synthesis, synthesis from optically pure precursors or by resolution of the racemates. Resolution of the racemates can be accomplished, for example, by conventional methods such as crystallization in the presence of a resolving agent, or chromatography, using, for example, a chiral HPLC column.

Compounds herein may also be described using a general formula that includes variables such as, *e.g.*, A R¹, R², R³, R⁴, R⁵, R⁶, R⁷, R¹⁰, R¹¹, R¹², R¹³, R¹⁴, R¹⁵, R²⁰, etc. Unless otherwise specified, each variable within such a formula is defined independently of any other variable, and any variable that occurs more than one time in a formula is defined independently at each occurrence. Also, combinations of substituents and/or variables are permissible only if such combinations result in stable compounds, *i.e.*, compounds that can be isolated, characterized and tested for biological activity.

A "pharmaceutically acceptable salt" of a compound disclosed herein preferably is an acid or base salt that is generally considered in the art to be suitable for use in contact with the tissues of human beings or animals without excessive toxicity or carcinogenicity, and preferably without irritation, allergic response, or other problem or complication. Such salts include mineral and organic acid salts of basic residues such as amines, as well as alkali or organic salts of acidic residues such as carboxylic acids. Suitable pharmaceutical salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC-(CH₂)ₙ-COOH where n is any integer from 0 to 4, *i.e.*, 0, 1, 2, 3, or 4, and the like. Similarly, pharmaceutically acceptable cations include, but are not limited to sodium, potassium, calcium, aluminum, lithium and ammonium. Those of ordinary skill in the art will recognize further pharmaceutically acceptable salts for the compounds provided herein. In general, a pharmaceutically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two. Generally, the use of nonaqueous media, such as ether, ethyl acetate, ethanol, isopropanol or acetonitrile, is preferred.

It will be apparent that each compound of formula (I) may, but need not, be present as a hydrate, solvate or non-covalent complex. In addition, the various crystal forms and polymorphs are within the scope of the present invention.

A "substituent," as used herein, refers to a molecular moiety that is covalently bonded to an atom within a molecule of interest, e.g. to a compound of general formula (I) or a prodrug thereof. For example, a "ring substituent" may be a moiety such as a halogen, alkyl group, haloalkyl group or other substituent described herein that is covalently bonded to an atom, preferably a carbon or nitrogen atom, that is a ring member. The term "substituted," as used herein, means that any one or more hydrogens on the designated atom is replaced with a selection from the indicated substituents, provided that the designated atom's normal valence is not exceeded, and that the substitution results in a stable compound, *i.e.*, a compound that can be isolated, characterized and tested for biological activity. When a substituent is oxo, *i.e.*, =O, then 2 hydrogens on the atom are replaced.

The expression alkyl refers to a saturated, straight-chain (or linear) or branched hydrocarbon group that contains from 1 to 20 carbon atoms, preferably from 1 to 12 carbon atoms, more preferably from 1 to 6 carbon atoms, for example a methyl, ethyl, propyl, iso-propyl, n-butyl, iso-butyl, *sec*-butyl, *tert-*butyl, n-pentyl, n-hexyl, 2,2-dimethylbutyl or n-octyl group.

The expressions alkenyl and alkynyl refer to at least partially unsaturated, straight-chain or branched hydrocarbon groups that contain from 2 to 20 carbon atoms, preferably from 2 to 12 carbon atoms, more preferably from 2 to 6 carbon atoms, for example an ethenyl, allyl, acetylenyl, propargyl, isoprenyl or hex-2-enyl group. Preferably, alkenyl groups have one or two, more preferably one, double bond(s) and alkynyl groups have one or two, more preferably one, triple bond(s).

The expression alkoxy refers to a saturated straight-chain or branched group of the general formula - OR, wherein R represents an alkyl group as defined above. An alkoxy group having 1 to 6 carbon atoms or 1 to 4 carbon atoms is preferred. Preferred examples of an alkoxy group having 1 to 6 carbon atoms include methoxy group, ethoxy group, n-propoxy group, isopropoxy group, n-butoxy group, isobutoxy group, s-butoxy group, t-butoxy group, n-pentyloxy group, n-hexyloxy group, and the like.

The expression "acyl group" refers to an alkylcarbonyl group, wherein the alkyl moiety is an alkyl group as described above. An acyl group having 2 to 6 carbon atoms or 2 to 4 carbon atoms is preferred. Examples of an acyl group include an acetyl group, a propanoyl group, 1-methylpropanoyl group, a butanoyl group, 1-methylpropanoyl group, 2-methylpropanoyl group, 1,1-dimethylpropanoyl group, a pentanoyl group, and the like. An acetyl group and a propanoyl group are mentioned as a preferred example.

The expression "halogen" or "halogen atom" as preferably used herein means fluorine, chlorine, bromine, iodine.

The expression "optionally substituted" as used in conncetion with any group, preferably refers to a group in which one or more hydrogen atoms have been replaced each independently of the others by fluorine, chlorine, bromine or iodine atom; or by OH, =O, SH, =S, NH₂, =NH, CN, NO₂, or an alkoxy group.

As for an optionally substituted alkyl group, a group in which one or more hydrogen atoms have been replaced each independently of the others by a hydroxyl group, a halogen atom, preferably a fluorine or chlorine atom, or a methoxy group can be mentioned as a preferred example. Additionally, an optionally substituted alkyl group may be one selected from the group consisting of the above described preferred examples of an alkyl group further including a trifluoromethyl group, a difluoromethyl group, a hydroxymethyl group, 2-hydroxyethyl group, and a methoxymethyl group. A methyl group, an ethyl group, a n-propyl group, an isopropyl group, a cyclopropyl group, a trifluoromethyl group, a difluoromethyl group, a hydroxymethyl group, a 2-hydroxyethyl group or a methoxymethyl group are more preferred as an optionally substituted alkyl group. As for a substituent for an optionally substituted alkenyl group, and a substituent for an optionally substituted alkynyl group, the substituents for an optionally substituted alkyl group as described above can be mentioned.

As used herein a wording defining the limits of a range of length such as, e. g., "from 1 to 5" means any integer from 1 to 5, i. e. 1, 2, 3, 4 and 5. In other words, any range defined by two integers explicitly mentioned is meant to comprise and disclose any integer defining said limits and any integer comprised in said range.

Preferred according to the present invention can be a compound represented by the general formula (I'), 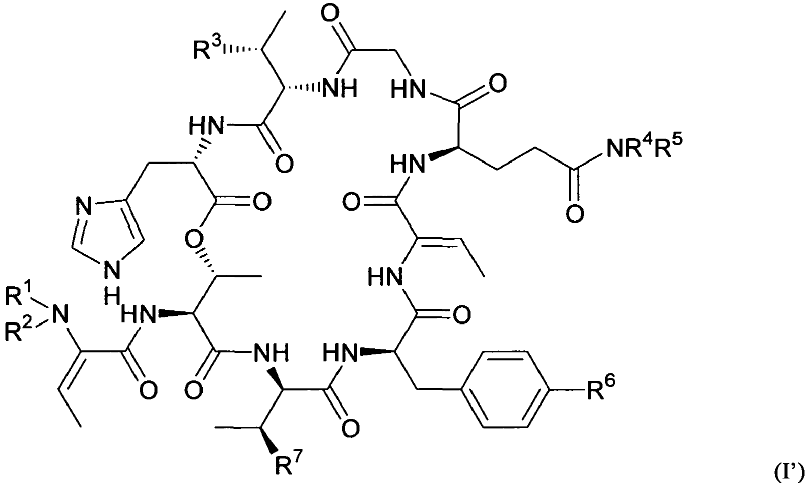 or a pharmacologically acceptable salt thereof; wherein R¹, R², R³, R⁴, R⁵, R⁶, R⁷ are defined as in general formula (I) above.

Preferably, R¹ can be a group represented by the general formula (II): 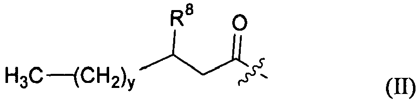 wherein
R⁸ can be a hydrogen atom, a halogen atom, a hydroxyl group, a cyano group, a nitro group, an amino group, a mercapto group, an optionally substituted alkyl group having 1 to 6 carbon atoms, an alkoxy group having 1 to 6 carbon atoms, a SOCH₃ group, a SO₂CH₃ group, an acyl group having 2 to 6 carbon atoms, or a polyethylene glycol group of formula -A-[CH₂-CH₂-O]ₙ-R²⁰, wherein A is -0-, -C(=O)-, -OC(=O)-, or -OC(=O)- (CH₂)ₘ-O-; m is an integer from 1 to 20; n is an integer of from 2 to 100, and R²⁰ is a hydrogen atom, a methyl group, or an ethyl group; more preferably R⁸ can be a halogen atom, a hydroxyl group, an optionally substituted alkyl group having 1 to 6 carbon atoms, an alkoxy group having 1 to 6 carbon atoms, a SOCH₃ group, a SO₂CH₃ group, or an acyl group having 2 to 6 carbon atoms; especially preferred R⁸ can be a halogen atom, a hydroxyl group, an amino group, or an optionally substituted alkyl group having 1 to 6 carbon atoms; and most preferred R⁸ can be a hydroxyl group; and
y is an integer from 1 to 20; more preferably an integer from 1 to 15; further preferred an integer from 1 to 10, and most preferred y is 10.

Preferably, R¹ can be a group represented by the general formula (II'): 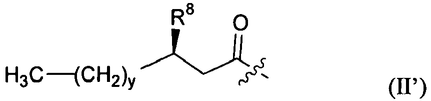 wherein R⁸ and y are defined as in general formula (II) above.

Also preferred, R² can be a hydrogen atom or an optionally substituted alkyl group having 1 to 6 carbon atoms; especially preferred R² represents a hydrogen atom or a methyl group; and most preferred R² can be a hydrogen atom.

Preferably, R³ can be a halogen atom, a hydroxyl group, an amino group, or an optionally substituted alkyl group having 1 to 6 carbon atoms; especially preferred R³ represents a halogen atom, a hydroxyl group, or an optionally substituted alkyl group having 1 to 6 carbon atoms; more preferably R³ can be a halogen atom, or a hydroxyl group; and most preferred R³ can be a hydroxyl group.

Preferably, R⁴ and R⁵ can each independently represent a hydrogen atom, or an optionally substituted alkyl group, wherein one carbon atom in said alkyl group may be replaced by an oxygen atom, a sulfur atom, C=O, NR¹³, CONR¹⁴, or NR¹⁵CO at any chemically allowable position; and R¹³, R¹⁴, and R¹⁵ are as defined above; especially preferred R⁴ and R⁵ can each independently represent a hydrogen atom, or an optionally substituted alkyl group; and most preferred R⁴ and R⁵ each represents a hydrogen atom.

Further preferred, R⁶ can be a halogen atom, a hydroxyl group, an amino group, an optionally substituted alkyl group having 1 to 6 carbon atoms, an alkoxy group having 1 to 6 carbon atoms, a SOCH₃ group, a SO₂CH₃ group, or an acyl group having 2 to 6 carbon atoms; especially preferred R⁶ can be a halogen atom, a hydroxyl group, an amino group, or an optionally substituted alkyl group having 1 to 6 carbon atoms; and most preferred R⁶ can be a hydroxyl group..

Preferably, R⁷ can be a halogen atom, a hydroxyl group, an amino group, an optionally substituted alkyl group having 1 to 6 carbon atoms, an alkoxy group having 1 to 6 carbon atoms, a SOCH₃ group, a SO₂CH₃ group, an acyl group having 2 to 6 carbon atoms, or a polyethylene glycol group of formula -A-[CH₂-CH₂-O]ₙ-R²⁰, wherein A -C(=O)-, or -OC(=O)- (CH₂)ₘ₋O-; m is an integer of from 1 to 6; n is an integer of from 2 to 10, and R²⁰ is a hydrogen atom, or a methyl group; especially preferred R⁷ can be a halogen atom, a hydroxyl group, an amino group, or an optionally substituted alkyl group having 1 to 6 carbon atoms; and most preferred R⁷ can be a hydroxyl group.

Especially preferred according to the invention can be a compound represented by formula (III): 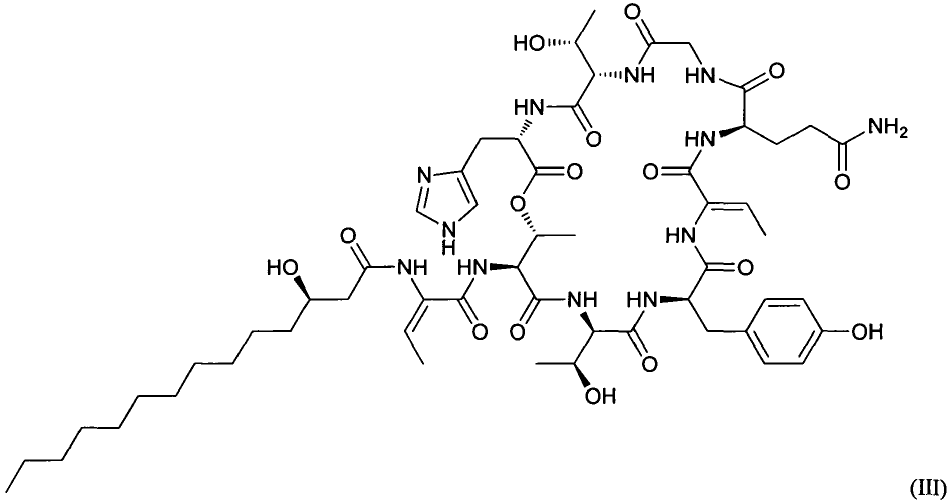 or a pharmacologically acceptable salt thereof.

The compound according to formula (III) may herein also be referred to as jagaricin.

It is to be noted that the present invention also encompasses all possible combinations of all preferred embodiments.

Compounds provided herein exhibit high antifungal activity with an inhibition constant (MIC) at nanomolar concentrations. Further, the compounds according to the invention may provide antitumor activity on cultured human tumor cell lines, i.e. an antiproliferative activity with an inhibition constant (GI₅₀) and/or a cytotoxic activity with an IC₅₀ or CC₅₀ in the micromolar range.

The activity and more specifically the pharmacological activity of the compounds according to the present invention can be assessed using appropriate *in vitro* assays. For instance, the GI₅₀, CC₅₀, or IC₅₀ values of the compounds according to the present invention may be determined via a cytotoxicity and antiproliferative assay of cell growth. Antifungal activities can, for example, be studied qualitatively by agar diffusion tests. Preferred compounds of the invention have values in the micromolar range, still more preferably values in the nanomolar range in the assays mentioned above.

Preferably, the compounds of formula (I) according to the present invention each have one or more pharmacological properties, especially, antiproliferative, antibacterial, antifungal or cytostatic activity, low toxicity, low drug drug interaction, high bioavailability, especially with regard to oral administration, high metabolic stability, and high solubility.

The therapeutic use of one or more compound(s) of formula (I), its/their pharmacologically acceptable salt(s) and also formulations and pharmaceutical compositions containing the same are within the scope of the present invention. The present invention also relates to the use of the compound of formula (I) as active ingredient in the preparation or manufacture of a medicament, especially, a compound of formula (I), its pharmacologically acceptable salt and also formulations and pharmaceutical compositions for use in the treatment of fungal infections or cancer as well as its/their use for the preparation of a medicament, particularly a medicament for the treatment of fungal infections or cancer.

The pharmaceutical compositions according to the present invention comprise at least one compound of formula (I) and, optionally, one or more carrier substances, excipients and/or adjuvants. Pharmaceutical compositions may additionally comprise, for example, one or more of water, buffers such as, *e.g.*, neutral buffered saline or phosphate buffered saline, ethanol, mineral oil, vegetable oil, dimethylsulfoxide, carbohydrates such as *e.g.*, glucose, mannose, sucrose or dextrans, mannitol, proteins, adjuvants, polypeptides or amino acids such as glycine, antioxidants, chelating agents such as EDTA or glutathione and/or preservatives. Furthermore, one or more other active ingredients may, but need not, be included in the pharmaceutical compositions provided herein. For instance, the compounds of the invention may advantageously be employed in combination with an antibiotic, another anti-fungal, or anti-viral agent, an-anti histamine, a non-steroidal anti-inflammatory drug, a disease modifying anti-rheumatic drug, another cytostatic drug, a drug with smooth muscle activity modulatory activity, or mixtures of the aforementioned.

Pharmaceutical compositions may be formulated for any appropriate route of administration, including, for example, topical such as, *e.g.,* transdermal or ocular, oral, buccal, nasal, vaginal, rectal or parenteral administration. The term parenteral as used herein includes subcutaneous, intradermal, intravascular such as, *e.g.,* intravenous, intramuscular, spinal, intracranial, intrathecal, intraocular, periocular, intraorbital, intrasynovial and intraperitoneal injection, as well as any similar injection or infusion technique. In certain embodiments, compositions in a form suitable for oral use are preferred. Such forms include, for example, tablets, troches, lozenges, aqueous or oily suspensions, dispersible powders or granules, emulsion, hard or soft capsules, or syrups or elixirs. Within yet other embodiments, compositions provided herein may be formulated as a lyophilizate.

Compositions intended for oral use may further comprise one or more components such as sweetening agents, flavoring agents, coloring agents and/or preserving agents in order to provide appealing and palatable preparations. Tablets contain the active ingredient in admixture with physiologically acceptable excipients that are suitable for the manufacture of tablets. Such excipients include, for example, inert diluents such as, *e.g.,* calcium carbonate, sodium carbonate, lactose, calcium phosphate or sodium phosphate, granulating and disintegrating agents such as, *e.g.,* corn starch or alginic acid, binding agents such as, *e.g.,* starch, gelatin or acacia, and lubricating agents such as, *e.g.,* magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monosterate or glyceryl distearate may be employed.

A composition may further include one or more components adapted to improve the stability or effectiveness of the applied formulation, such as stabilizing agents, suspending agents, emulsifying agents, viscosity adjusters, gelling agents, preservatives, antioxidants, skin penetration enhancers, moisturizers and sustained release materials. Examples of such components are described in Martindale--The Extra Pharmacopoeia (Pharmaceutical Press, London 1993) and Martin (ed.), Remington's Pharmaceutical Sciences.

For the treatment of fungal infections as well as for the treatment of cancer, the dose of the biologically active compound according to the invention may vary within wide limits and may be adjusted to individual requirements. The required dose may be administered as a single dose or in a plurality of doses. The amount of active ingredient that may be combined with the carrier materials to produce a single dosage form will vary depending upon the host treated and the particular mode of administration. Dosage unit forms will generally contain a sufficient amount of active ingredient. It will be understood, however, that the specific dose level for any particular patient will depend upon a variety of factors including the activity of the specific compound employed, the age, body weight, general health, sex, diet, time of administration, route of administration, and rate of excretion, drug combination, *i.e.* other drugs being used to treat the patient, and the severity of the particular disease undergoing therapy.

Preferred compounds of the invention will have certain pharmacological properties. Such properties include, but are not limited to oral bioavailability, such that the preferred oral dosage forms discussed above can provide therapeutically effective levels of the compound *in vivo*.

Compounds provided herein are preferably administered to a patient such as, *e.g.*, a human, orally or topically, and are present within at least one body fluid or tissue of the patient. Accordingly, further disclosed are methods for treating patients suffering from a fungal disease or cancer. As used herein, the term "treatment" encompasses both disease-modifying treatment and symptomatic treatment, either of which may be prophylactic, *i.e.*, before the onset of symptoms, in order to prevent, delay or reduce the severity of symptoms, or therapeutic, *i.e.*, after the onset of symptoms, in order to reduce the severity and/or duration of symptoms. Patients may include but are not limited to primates, especially humans, domesticated companion animals such as dogs, cats, horses, and livestock such as cattle, pigs, sheep, with dosages as described herein.

The compounds of the present invention are useful in the treatment of different cancers, such as, for example, breast, colon, lung and prostate tumors, as well as osteosarcoma, acute myeloid leukaemia, sporadic endometrial cancer, melanoma, malignant melanoma, soft tissue Sarcoma, B-cell chronic lymphocytic leukaemia, gastric cancers, cervical cancer, hepatocellular carcinoma, pancreatic cancer; renal cancer/kidney cancer, or colorectal cancer.

Also the following methods for producing a compound of formula (I) lie within the scope of the present invention.

The development of natural product based drugs is often hampered by their structural complexity. This fact precludes facile total synthetic access to analogues or the development of natural product libraries. Therefore, semisynthetic as well as biotechnological approaches are commonly pursued in pharmaceutical research and development (von Nussbaum et al., Angew. Chem. Int. Ed. 2006, 45, 5072-5129). A very interesting strategy combines chemical semisynthesis with biosynthesis using genetically engineered microorganisms, a technique which occasionally has been termed mutational biosynthesis or in short mutasynthesis (Review: S. Weist, R. D. Süssmuth, Appl. Microbiol. Biotechnol. 2005, 68, 141-150).

For instance, a compound of formula (I), e.g. jagaricin, can be produced by culturing *Janthinobacterium agaricidamnosum* (DSM 9628). It is understood that the production of compounds of formula (I) is not limited to the use of the particular organism described herein, which is given for illustrative purpose only. The invention also includes the use of any mutants which are capable of producing a compound of formula (I) including natural mutants as well as artificial mutants, e.g. genetically manipulated mutants and the expression of the gene cluster responsible for biosynthesis in a producer strain or by heterologous expression in host strains.

A compound of formula (I) can be produced in liquid culture, by growing the respective microorganism in media containing one or several different carbon sources, and one or different nitrogen sources. Also salts are essential for growth and production. Suitable carbon sources are different mono-, di-, and polysaccharides like maltose, glucose or carbon from amino acids like peptones. Nitrogen sources are ammonium, nitrate, urea, chitin or nitrogen from amino acids. The following inorganic ions support the growth or are essential in synthetic media: Mg-ions, Ca-ions, Fe-ions, Mn-ions, Zn-ions, K-ions, sulfate-ions, Cl-ions, phosphate-ions.

Temperatures for growth and production are between 10°C to 40 °C, preferred temperatures are between 20 °C and 30 °C, especially at 22 °C and 28 °C, respectively. The pH of the culture solution is from 5 to 8, preferably 6.5 and 7.5.

A compound of formula (I) can also be obtained by chemical synthesis using usual chemical reactions and synthesis methods known to a person skilled in the art.

### Brief Description of the Figures

***Figure 1******.*** Biosynthesis gene cluster with structure of jagaricin as well as the modular organisation of the jagaricin synthetase.
***Figure 2******.*** HPLC chromatogram of *J. agaricidamnosum ΔjagA* extract, the wild type extract and medium extract. The jagaricin peak is framed.
*Figure 3**.* Plasmide map of pKG01. MCS-multi cloning site; AmpR-Ampicillin resistance cassette; KmR-Kanamycin resistance cassette.

The present invention is now further illustrated by the following examples from which further features, embodiments and advantages of the present invention may be taken.

### EXAMPLES

All reagents were purchased from commercial suppliers and used without further purification. Additionally, experiments were usually performed according to standard protocols and according to the manufacturer's protocol, respectively. Specific methods and materials are summarized below.

### Bacterial strains and media

*Janthinobacterium agaricidamnosum* (DSM 9628) was retrieved from the German collection of microorganisms (DSM). The bacteria were cultured in nutrient media (605 DSM without NaCl; 1 g/L beef extract, 2 g/L yeast extract, 5 g/L peptone, 15 g/L agar). Plates and liquid cultures (shaken at 150 rpm in baffled flasks) were grown at 22 °C and 28 °C, respectively. During screening plates and liquid cultures of modified nutrient agar (additional 4 g/L chitin, 100 g/L mushroom cubes), M9 (6 g/L Na₂HPO₄, 3 g/L KH₂PO₄, 0.5 g/L NaCl, 2 g/L NH₄Cl, 4 g/L glucose, 25 g/L FeSO₄, 2 mM MgSO₄, 15 g/L agar), MS (20 g/L mannite, 20 g/L soy flour, 20 g/L agar) and modified VK (5 g/L glycine, 10 g/L yeast extract, 10 g/L glucose, 10 g/L corn steep, 10 g/L CaCO₃, 15 g/L agar pH 6.7-7.0) media were used. For the production of jagaricin *J. agaricidamnosum* was grown in modified VK medium. The selection of positive *J. agaricidamnosum* mutants was carried out on nutrition agar supplemented with 50 µg/mL kanamycin. For the construction of pKG01, *Escherichia coli* Top 10 cells and *E. coli* ER2925 cells were used. *E. coli* was cultured in LB medium (10 g/L tryptone, 5 g/L yeast extract, 5 g/L NaCl, 1 g/L glucose) supplemented with 50 µg/mL kanamycin. *Candida albicans, Aspergillus fumigatus* and *A. terreus* were used for the bioactivity tests and they were cultivated on malt agar (C. *albicans*; 40 g/L malt extract, 4 g/L yeast extract, 15 g/L agar, pH 5.7-6.0) and potato glucose agar (*A. fumigatus* and *A. terreus*; 4 g/L potato starch infusion, 20 g/L dextrose, 15 g/L agar) plates, respectively.

### Screening for secondary metabolites

Bacterial 20 mL cultures were grown for three days. Then the cultures were extracted twice with 20 mL of ethyl acetate. Next, the ethyl acetate was removed under reduced pressure. The residue was dissolved in 0.5 mL methanol and was analyzed via analytical HPLC (Shimadzu LC-10Avp series with autosampler, high pressure pumps, column oven and DAD detector, C18 column (Eurospher 100-5 250x4.6 mm), 1 mL/min flow rate, gradient elution (MeCN/0.1 % TFA 0.5/99.5 to 100/0 within 30 minutes)) and mass spectrometry measurements (direct injection of 10 µL; Exactive, Thermo Scientific).

### Expression analysis

RNA isolation was carried out with TRIsure RNA isolation reagent (Bioline). Thereby, the pellet of a 1 mL overnight culture was resuspended in 1.5 mL reagent. Cell disruption at SpeedMill PLUS (analytikjena) was carried out in lysis tubes (Biospec Products). Subsequently, remaining DNA was removed with Turbo-DNAse (Ambion). A one step RT-PCR kit (One Step SYBR PrimeScript RT-PCR Kit, TaKaRa) and commercially purchased primers were used for reverse transcription (42 °C 5 min, 95 °C 10 sec) and amplification (95 °C 5 sec, 54 °C 10 sec, 72 °C 15 sec; cycle was repeated 40 times).

### Purification of jagaricin

The crude extract of a 50 L fermentation was separated by size exclusion chromatography (Sephadex-LH-20 column with methanol as mobile phase). An additional purification step was carried out via preparative HPLC (Shimadzu LC-8a series, DAD detector, C-18 column Grom-Saphir-110C (250x20 mm), 10 mL/min flow rate, 83 % MeCN/0.01 % TFA 10/90 to 100/0 within 25 minutes) and yielded the pure compound.

### Structural elucidation

The ester bond of the lipopeptide was hydrolyzed by incubation in 1 M NaOH at room temperature for 1 hour. After the neutralization, MS/MS analyses were carried out with the linearized and with the unmodified compound using the TSQ Quantum (Thermo Scientific) and the Exactive (Thermo Scientific) MS instrument in order to get information about the amino acid sequence. For NMR measurements jagaricin was dissolved in deuterated methanol. NMR spectra were recorded on Bruker Avance DRX 500 and DRX 600 instruments (**Table S2**, S11-16). Spectra were referenced to the residual solvent signals.

**Table S2. NMR shifts of β-hydroxymyristic acid and the amino acids starting from the N-terminus.**

| **partial structure** | **position** | **¹³C NMR** | **¹H NMR (mult., *J* in Hz)** |
|---|---|---|---|
| β-hydroxymyristic acid | | | |
| | 1 | 172.8 | - |
| | 2 | 44.7 | 2.39 (1H, *) |
| | | | 2.34 (1H, d, 8.6) |
| | 3 | 69.8 | 3.94 (1H, m) |
| | 4 | 38.4 | 1.45 (2H,*) |
| | 5 | 26.6 | 1.44 (1H,*) |
| | | | 1.29 (1H,*) |
| | 6-11 | 26.6-30.8 (6C) | 1.24-1.35 (12H,*) |
| | 12 | 33.1 | 1.27 (2H,*) |
| | 13 | 23.7 | 1.29 (2H,*) |
| | 14 | 14.5 | 0.89 (3H, t, 6.9) |
| | 3-OH | - | n. d. |
| dehydrobutyrine | | | |
| | 1 | 168.3⁺ | - |
| | 2 | 132.2 | - |
| | 3 | 118.7 | 5.71 (1H, q, 7.3) |
| | 4 | 13.8 | 1.87 (3H, d, 7.3) |
| | NH | - | n. d. |
| L-threonine | | | |
| | 1 | 171.1 | - |
| | 2 | 58.8 | 4.71 (1H,d, 4.1) |
| | 3 | 72.3 | 5.50 (1H, brs) |
| | 4 | 17.0 | 1.34 (3H, d, 6.5) |
| | NH | - | n. d. |
| D-threonine | | | |
| | 1 | 172.8 | - |
| | 2 | 60.6 | 4.34 (1H,*) |
| | 3 | 68.7ⁱ | 4.10 (1H,*) |
| | 4 | 20.7 | 1.18 (3H, t, 5.9*) |
| | NH | - | n. d. |
| D-tyrosin | | | |
| | 1 | 173.9 | - |
| | 2 | 57.3 | 4.37 (1H,*) |
| | 3 | 36.9 | 3.24 (1H, dd,**) |
| | | | 2.99 (1H, dd, 14.4,9.2) |
| | 4 | 128.9 | - |
| | 5,9 | 131.3 | 7.09 (2H, d, 8.4) |
| | 6,8 | 116.4 | 6.68 (2H, d, 8.4) |
| | 7 | 157.4 | - |
| | 7-OH | - | n. d. |
| | NH | - | n. d. |
| dehydrobutyrine | | | |
| | 1 | n.d. | - |
| | 2 | 131.2ⁱ | - |
| | 3 | 130.8⁺ | 6.39 (1H, brs) |
| | 4 | 13.2 | 1.60 (3H, d, 6.9) |
| | NH | - | n. d. |
| D-glutamine | | | |
| | 1 | 174.4 | - |
| | 2 | 56.1 | 4.29 (1H, dd,**) |
| | 3 | 27.9 | 2.13 (2H, m) |
| | 4 | 32.8 | 2.39 (2H,*) |
| | 5 | 178.0 | - |
| | 5-NH₂ | - | n. d. |
| | NH | - | n. d. |
| Glycine | 1 | 172.3 | - |
| | 2 | 44.0 | 4.02 (1H, d, 16.6) |
| | | | 3.80 (1H, d, 16.6) |
| | NH | - | n. d. |
| L-threonine | | | |
| | 1 | 172.4 | - |
| | 2 | 61.3 | 4.15 (1H, d, 5.1) |
| | 3 | 68.3ⁱ | 4.08 (1H,*) |
| | 4 | 20.0 | 1.18 (3H, t, 5.9*) |
| | NH | - | n. d. |
| L-histidine | | | |
| | 1 | 170.3 | - |
| | 2 | 54.1 | 4.56 (1H, t, 7.5) |
| | 3 | 27.4 | 3.03 (1H,*) |
| | | | 2.91 (1H,*) |
| | 4 | 131.1ⁱ | - |
| | 5 | 135.0 | 8.61 (1H, s) |
| | 6 | 119.0 | 7.35 (1H, s) |
| | 5-NH | - | n. d. |
| | NH | - | n. d. |

| | | | |
|---|---|---|---|
| * partial overlapping n. d. not detected ⁺ deduced from 2D couplings ⁱ interchangeable signals ** coupling was observed, but coupling constant not determined | | | |

The amino acid stereochemistry was determined by derivatisation with 1-fluoro-2,4-dinitrophenyl-5-L-alanine-amide (L-FDAA). First, jagaricin was hydrolyzed with 6 M HCl supplemented with 0.05 % phenol overnight at 105 °C. The solvent was removed by reduced pressure and 100 µL 1 M NaHCO₃ along with 50 µL L-FDAA (10 mg/mL in acetone) were added to the reaction that was heated at 50 °C for 1 h. Next, 50 µL 2 M HCl were added and the reaction mixture was diluted with 200 µL 50 % (vol/vol) acetonitrile. The derivatives were analyzed via analytical HPLC (Shimadzu LC-10Avp series, DAD detector, column Grom-Sil 100 ODS-0 AB, 3 µm (250x4.6 mm), 1 mL/min flow rate, MeCN/0.1 % TFA 25/75 to 60/40 within 35 minutes to 100/0 within 8 minutes). The retention times (minutes) of standard amino acids were as follows: D-His, 5.23; L-His, 6.47; L-Thr, 11.57; L-Gln, 14.4; D-Thr, 15.57; D-Gln, 15.61; L-Tyr, 34.34; D-Tyr, 37,36; jagaricin, L-His, 6.47; jagaricin, D-Thr, 11.57; jagaricin, D-Gln, 15.61, jagaricin, D-Tyr, 37,36. D-Thr, D-Gin, L-*allo-*Thr and D-*allo*-Thr were additionally analyzed as previously described with the altered gradient (25/75 to 57/43 within 35 minutes to 70/30 within 10 minutes to 100/0 within 5 minutes). The retention times (minutes) of standard amino acids were as follows: L-*allo*-Thr, 11.66; *D-allo*-Thr, 12.26; D-Thr, 15.57; D-Gln, 16.25; jagaricin, D-Thr, 15.48; jagaricin, D-Gln, 16.25. Additionally, the free β-hydroxy-myristic acid (HMA) obtained from hydrolysis and the (R)-and (S)-HMA standards (both TRC) were derivatized after hydrolysis of jagaricin with (R)-(-)-α-methoxy-a-trifluoromethylphenylacetyl chloride reagent as previously described (Jenske, R. and W. Vetter, J. Chromatogr. A., 2007. 1146(2): p. 225-31) whereby the methylation of the fatty acid was carried out in hexan/methanol with trimethylsillyldiazomethan at room temperature for 10 minutes. For the subsequent reaction 4-dimethylaminopyridine was used instead of pyridine. For GC-MS measurements the samples were dissolved in methanol. The analytics were executed on a Thermo Trace GC Ultra coupled with a FID and a Thermo Polaris Q electron impact (EI)-ion trap mass spectrometer equipped with Combi PAL autosampler. A SGE forte capillary column BPX5 30 m; 0.25 mm inner diameter and 0.25 µm film was used. The column was operated with helium carrier gas 1.0 mL/min and splitless injection. Injector temperature was 300°C, splitless time 1.0 min, then split flow was set to 15 mL/min. The FID temperature were set to 250°C with 35 mL/min hydrogen, 350 mL/min synthetic air and 30 mL/min helium make up gas. Method was carried out like previously described (Jenske and Vetter 2007, *loc.cit.*). Total ion current (TIC) were obtained using the mass range of 50-600 amu. 10 µL sample were injected into the GC.

### Acetylation of jagaricin

9 mg of substance was incubated with 1 mL pyridine (water free) and 1 mL acetic anhydrid over night under light exclusion. The acetylated product was precipitated with 20 mL ice cold water. Subsequently the substance was extracted three times with 20 mL chloroform. The organic phase was washed two times with distilled water and dried with Na₂SO₄. After removing the solvents under reduced pressure, the product was analyzed by analytical HPLC and afterwards the acetylated jagaricin was subjected to NMR measurements.

### Analysis of C-domain in module 5

The amino acid sequence of core motifes C 1 through C 7 were manually compared with the ones of ^{L}C_{L}- and ^{D}C_{L}-domains that were analyzed by Rausch and co-workers (Rausch, C. et al., BMC Evol. Biol., 2007; 7: p. 78). Additionally, a phylogeny of all C-domains of the jagaricin synthetase was constructed (data not shown). Alignment and tree construction were performed with Mega 3.1 (Molecular Evolutionary Genetics Analysis, Version 3.1, Kumar, Tamura and Nei).

### Bioactivity tests

50 µL of different concentrated jagaricin solutions were each pipetted into a pierced hole (9 mm diameter) within an agar plate that was inoculated with the test strains *C. albicans, A. fumigatus* and *A. terreus*, respectively. After incubation at 24 °C over night the inhibition zone was measured. The antiproliferative and cytotoxic assay were performed as previously described (Abdou, R. et al., Phytochemistry, 2009. 71(1): p. 110-6).

### Annotation of the jagaricin biosynthesis gene cluster

The *J. agaricidamnosum* genome was visualized with Artemis (Rutherford, K. et al., Bioinformatics, 2000. 16(10): p. 944-5) and manually scanned for long open reading frames which are likely to belong to natural product biosynthesis gene clusters. The corresponding amino acid sequences were analyzed via NCBI BLAST (Altschul S.F. et al., J. Mol. Biol., 1990. 215(3): p. 403-10; Sayers, E.W. et al., Nucleic. Acids Res., 2011. 39(Database issue): p. D38-51) and the PKS/NRPS analysis web site (Bachmann, B.O. and J. Ravel, Methods Enzymol, 2009. 458: p. 181-217) in order to search for conserved NRPS and PKS domains. The NRPSpredictor2 (Rottig, M. et al., Nucleic. Acids Res., 2011. 39(Web Server issue): p. W362-7) was used to characterize the A-domain specificity.

### Phylogenetic tree construction of thioesterase domains

Amino acid sequences from thioesterase (Te) domains of cyclic lipopeptides were obtained from the ClustScan data base (Starcevic, A. et al., Nucleic. Acids Res., 2008. 36(21): p. 6882-92). Alignment and tree construction (data not shown) were performed with Mega 3.1 (Molecular Evolutionary Genetics Analysis, Version 3.1, Kumar, Tamura and Nei).

### Construction of pKG01

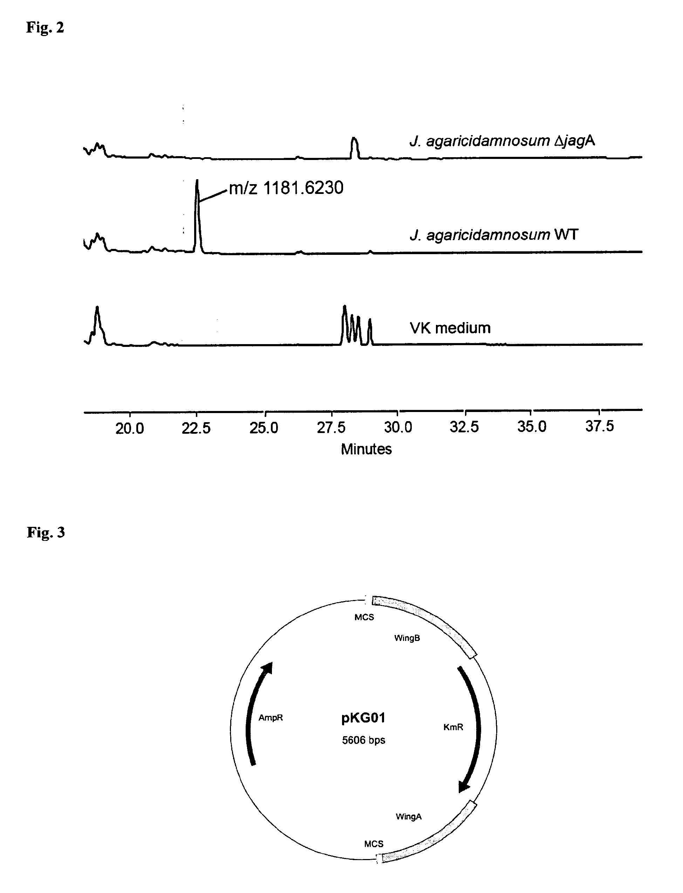

800 bp long homologous regions up- and downstream from the C₁-domain coding region were amplified via PCR using appropriate forward and reverse primer pairs. Additionally, the kanamycin resistance cassette was amplified from the template pK19 by employing an appropriate primer pair that incorporates a 30 bp overhang which is homologous to the above primer pairs for amplifying the C₁-domain coding region. The Taq polymerase (NEB) carried out all amplification reactions. The three PCR products were subjected to an overlapping PCR. For this reaction Phusion Flash PCR master mix (Thermo Scientific) and the appropriate pair were used. The product was cut with *Pst*I and subsequently ligated into the with *Pst*I cut pGem-T Easy (Promega), yielding the plasmide pKG01 **(****Figure 3****).** Next, the plasmide was used to transform *E. coli* ER2925.

### Transformation of J. agaricidamnosum

20 mL of nutrition media was inoculated with 0.5 mL of a bacterial overnight culture. The flask was shaken until OD₆₀₀ reached 0.3. All subsequent steps were carried out on ice. Cells were harvested by centrifuging for 5 min at 5,000 rpm at 4 °C. The pellet was washed two times with 20 mL and 10 mL 300mM sucrose, respectively. Cells were dissolved in 0.5 mL 300 mM sucrose. 1 µL of demethylated plasmide was added to an aliquot of 60 µL competence cells and electroporation was carried out (2 mm cuvette, 2500 V, 25 µF, 200 Ω). Cells were shaken at 25 °C for 1-2 h for recovery and plated on nutrition agar supplemented with 50 µg/mL kanamycin. The resulting mutants were checked via PCR. In addition, the ability to produce jagaricin was tested as previously described ('Screening for secondary metabolites').

### Imaging mass spectrometry

All mentioned chemical compounds, soft- and hardware were purchased by Bruker Daltonics. Commercial mushroom fruit bodies (*Agaricus bisporus*) were cut into approx. 1 mm thin slices that were placed on a conductive glass slide covered with double-sided adhesive coal tape (Plano). The mushroom tissue was inoculated with an overnight culture of *J. agaricidamnosum* and with jagaricin dissolved in water, respectively. The sample was incubated at room temperature for 24 h under moist conditions. Next, the sample was treated with a saturated solution of α-cynano-4-hydroxy cinnamic acid dissolved in a 2:1 mixture of 0.1 % TFA/MeCN. After a final drying step at 37 °C for 2 hours, the glass slide was clamped into a MTP slide adapter II and was subjected to MALDI-MS measurements. For this purpose a ultrafleXtreme mass spectrometer was used operating with flexControl 3.0 in positive reflector mode collecting data in the range of *m*/*z* 900-2000 Da. The laser intensity was set to 80% with a laser frequency of 1000 Hz. Before the run, the flexControl method was calibrated using peptide calibration standard II. The automatic scanning of the imaging area was programmed in flexImaging 3.0 with a raster width of 100 µm in XY recording 1000 spectra with a sample rate of 2 GS/s at every spot. The resultant sum spectrum was evaluated manually and the mass of interest was visualized in the logarithmic scale by picking the peak with 1 Da mass range using the brightness optimization as implemented in flexImaging.

### Example 1: Assessing the annotated natural product biosynthesis gene cluster in the genome of J. agaricidamnosum

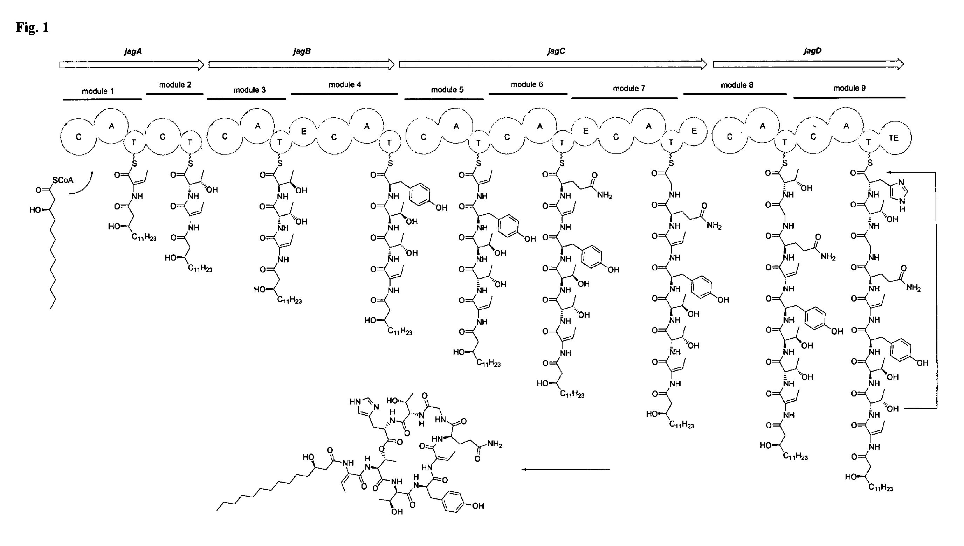

One gene cluster coding for a nine modular NRPS was assigned as the potential jagaricin biosynthesis gene cluster **(****Figure 1****),** as the size of the potential product matched the molecular weight of the isolated compound. Additionally, expression analyses of the biosynthesis gene cluster supported this assumption, as they showed an expression of the gene cluster during growth in producing media but not in non-producing media.

The jagaricin synthetase shows some interesting features. The first module exhibits a starter C-domain, that catalyses the condensation of a CoA-activated fatty acid with the first amino acid, leading to the biosynthesis of a lipopeptide. Also it is noteworthy, that the glycine activating module number seven possesses an E-domain, although glycine is not a chiral amino acid. However, E-domains do not only have their catalytic function, but they are also important for protein-protein interaction between NRPS subunits. Since the described E-domain is located at the C-terminus of JagC, a structural important role for this domain is very likely. Another noteable feature of the jagaricin biosynthesis gene cluster is the missing A-domain in module number two. A similar domain organization has been described for the yersiniabactin synthetase. During the synthesis of the siderophore yersiniabactin the second A-domain loads, in addition to the T-domain in the same module, two additional T-domains. Therefore, the loading of the second Thr in the jagaricin biosynthesis is probably carried out by the first A-domain.

Employing the modular structure of the biosynthesis gene cluster, the assigned A-domain specificities and MS/MS analyses, we were able to predict that jagaricin is a cyclic lipopeptide with the amino acid sequence C₁₄H₂₆O₂-Dhb-Thr-Thr-Tyr-Dhb-Gln-Gly-Thr-His **(****Figure 1****).** The ring closure was expected to lie in between His and the first Thr. NMR studies confirmed the predicted structure of jagaricin and identified the fatty acid chain as β-hydroxy-myristic acid. However, no couplings were observed in 2D NMR experiments that could verify the position of the ring closure. Yet, a phylogenetic analysis of Te-domains of different cyclic lipopeptides supported the proposed position for the ring closure. Further NMR experiments with OH-acetylated jagaricin could validate the ring closure position. The absolute stereochemistry was elucidated by using derivatisation reactions with Marfey's and Mosher's reagent, respectively **(****Figure 1****).** Thereby, it was at first surprising to identify D-Tyr instead of L-Tyr, as there is no epimerization domain in the fourth module. However, detailed analysis of the C-domain in the downstream module revealed the signature sequences of a ^{D}C_{L}-domain. Hence, the upstream A-domain activates probably D-Tyr that is supplied by an in *trans* acting racemase. This D-Tyr is subsequently build into the growing peptide chain via the ^{D}C_{L}-domain. Several examples where D-amino acids are incorporated into NRPs via this mechanism have been studied in the past. Thus, the stereochemistry of the amino acids coincided with the modular architecture of the jagaricin synthetase.

### Example 2: Assessing the bioactivity of jagaricin

Bioactivity studies showed strong antifungal activity of jagaricin against the major human pathogens *Candida albicans, Aspergillus fumigatus* and *Aspergillus terreus* **(Table 1)**, but little or no antibacterial activity (data not shown). In higher concentrations jagaricin exhibits antiproliferative and cytotoxic activity **(Table 1).**

**Table 1. Biological activity of jagaricin [µM].**

| | Ate | Afu | Cal | HUVEC | K-562 | HeLa |
|---|---|---|---|---|---|---|
| MIC | 0.28 | 0.41 | 0.42 | - | - | - |
| GI₅₀ | - | - | - | 1 | 1 | - |
| CC₅₀ | - | - | - | - | - | 3.8 |

| | | | | | | |
|---|---|---|---|---|---|---|
| Ate: *A. terreus;* Afu: *A. fumigatus;* Cal: C. *albicans* | | | | | | |

### Example 3: Assessing the involvement of jagaricin in the soft rod infection process

Imaging mass spectrometry studies could visualize the production of jagaricin within the damaged tissue (data not shown). Moreover, appliance of purified jagaricin also caused a superficial lesion on mushroom tissue. In order to evaluate the biological function of jagaricin further and to validate the annotation of the jagaricin biosynthesis gene cluster, the jagaricin biosynthesis gene cluster was disrupted by insertion of a kanamycin resistance cassette. The correct insertion of the kanamycin resistance cassette was checked via PCR. The knock-out mutant *Δjag*A showed neither jagaricin production in production media VK (**Figure 2**) nor on mushroom tissue. Therefore, the knock-out provides evidence, that jagaricin biosynthesis gene cluster was correctly annotated. Though, the mutant was still able to cause lesions on the mushroom fruit bodies.

These results indicate, that although jagaricin is involved in the infection process, it is not essential for pathogenicity. Thus, enzymes take part in the degradation process of the fruit bodies, as well. Studies of the brown blotch disease identified tolaasin as the sole virulence factor, while degradation enzymes have been shown to be the only virulence factor in the cavity disease caused by Burkholderia gladioli pv. agaricicola. However, the discovered mode of action, where produced toxins are not essential for pathogenicity, but contribute to the disease outcome, has also been described for the plant pathogen Pseudomonas syringae.

## Claims

1. A compound of the general formula (I): 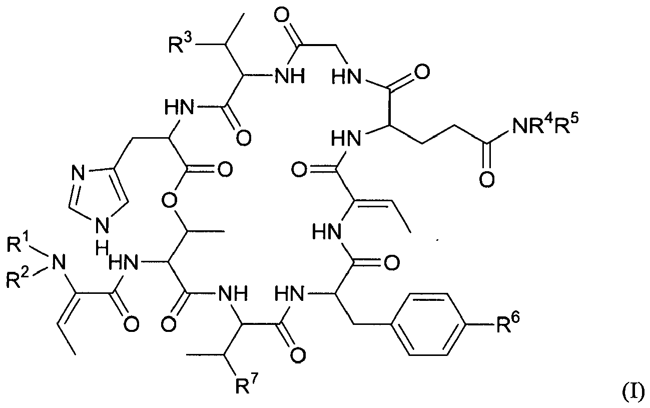 or a pharmacologically acceptable salt thereof, wherein
R¹ and R² each independently represents a hydrogen atom, an alkyl group in which one or more hydrogen atoms optionally have been replaced each independently of the others by a fluorine, chlorine, bromine or iodine atom: or by OH, =O, SH. =S, NH₂, =NH, CN, NO₂, or an alkoxy group, an alkenyl group in which one or more hydrogen atoms optionally have been replaced each independently of the others by a fluorine, chlorine, bromine or iodine atom: or by OH, =O, SH, =S, NH₂, =NH, CN, NO₂, or an alkoxy group, or an alkinyl group in which one or more hydrogen atoms optionally have been replaced each independently of the others by a fluorine, chlorine, bromine or iodine atom: or by OH, =O, SH, =S, NH₂, =NH, CN, NO₂, or an alkoxy group, and wherein one carbon atom in said alkyl, alkenyl, or alkynyl group may be replaced by an oxygen atom, a sulfur atom, C=O, NR¹⁰, CONR¹¹, or NR¹²CO at any chemically allowable position;
R¹⁰, R¹¹, and R¹² each independently represents a hydrogen atom or an alkyl group having 1 to 6 carbon atoms;
R³ is a hydrogen atom, a halogen atom, a hydroxyl group, a cyano group, a nitro group, an amino group, a mercapto group, an alkyl group having 1 to 6 carbon atoms in which one or more hydrogen atoms optionally have been replaced each independently of the others by a fluorine, chlorine, bromine or iodine atom; or by OH, =O, SH, =S, NH₂, =NH, CN, NO₂, or an alkoxy group, an alkoxy group having 1 to 6 carbon atoms, a SOCH₃ group, a SO₂CH₃ group, or an acyl group having 2 to 6 carbon atoms, or a polyethylene glycol group of formula -A-[CH₂-CH₂-O]ₙ-R²⁰, wherein A is -O-, -C(=O)-, -OC(=O)-, or -OC(=O)- (CH₂)ₘ-O-; m is an integer from 1 to 20; n is an integer of from 2 to 100, and R²⁰ is a hydrogen atom, a methyl group, or an ethyl group;
R⁴ and R⁵ each independently represents a hydrogen atom, an alkyl group in which one or more hydrogen atoms optionally have been replaced each independently of the others by a fluorine, chlorine, bromine or iodine atom; or by OH, =O, SH, =S, NH₂, =NH, CN, NO₂, or an alkoxy group, an alkenyl group in which one or more hydrogen atoms optionally have been replaced each independently of the others by a fluorine, chlorine, bromine or iodine atom: or by OH, =O, SH, =S, NH₂, =NH, CN, NO₂, or an alkoxy group, or an alkinyl group in which one or more hydrogen atoms optionally have been replaced each independently of the others by a fluorine, chlorine, bromine or iodine atom; or by OH, =O, SH, =S, NH₂, =NH, CN, NO₂, or an alkoxy group, wherein one carbon atom in said alkyl, alkenyl, or alkynyl group may be replaced by an oxygen atom, a sulfur atom, C=O, NR¹³, CONR¹⁴, or NR¹⁵CO at any chemically allowable position;
R¹³, R¹⁴, and R¹⁵ each independently represents a hydrogen atom or an alkyl group having 1 to 6 carbon atoms;
R⁶ is a hydrogen atom, a halogen atom, a hydroxyl group, a cyano group, a nitro group, an amino group, a mercapto group, an alkyl group having 1 to 6 carbon atoms in which one or more hydrogen atoms optionally have been replaced each independently of the others by a fluorine, chlorine, bromine or iodine atom; or by OH, =O, SH, =S, NH₂ =NH, CN, NO₂, or an alkoxy group, an alkoxy group having 1 to 6 carbon atoms, a SOCH₃ group, a SO₂CH₃ group, an acyl group having 2 to 6 carbon atoms, or a polyethylene glycol group of formula -A-[CH₂-CH₂-O]ₙ-R²⁰, wherein A is -0-, -C(=O)-, -OC(=O)-, or -OC(=O)- (CH₂)ₘ-O-; m is an integer from 1 to 20; n is an integer of from 2 to 100, and R²⁰ is a hydrogen atom, a methyl group, or an ethyl group; and
R⁷ is a hydrogen atom, a halogen atom, a hydroxyl group, a cyano group, a nitro group, an amino group, a mercapto group, an alkyl group having 1 to 6 carbon atoms in which one or more hydrogen atoms optionally have been replaced each independently of the others by a fluorine, chlorine, bromine or iodine atom; or by OH, =O, SH, =S, NH₂, =NH, CN, NO₂, or an alkoxy group, an alkoxy group having 1 to 6 carbon atoms, a SOCH₃ group, a SO₂CH₃ group, or an acyl group having 2 to 6 carbon atoms, or a polyethylene glycol group of formula -A-[CH₂-CH₂-O]ₙ-R²⁰, wherein A is -O-, -C(=O)-, -OC(=O)-, or -OC(=O)- (CH₂)ₘ-O-; m is an integer from 1 to 20; n is an integer of from 2 to 100, and R²⁰ is a hydrogen atom, a methyl group, or an ethyl group.

2. The compound according to claim 1 or a pharmacologically acceptable salt thereof, wherein the compound is represented by the general formula (I'): 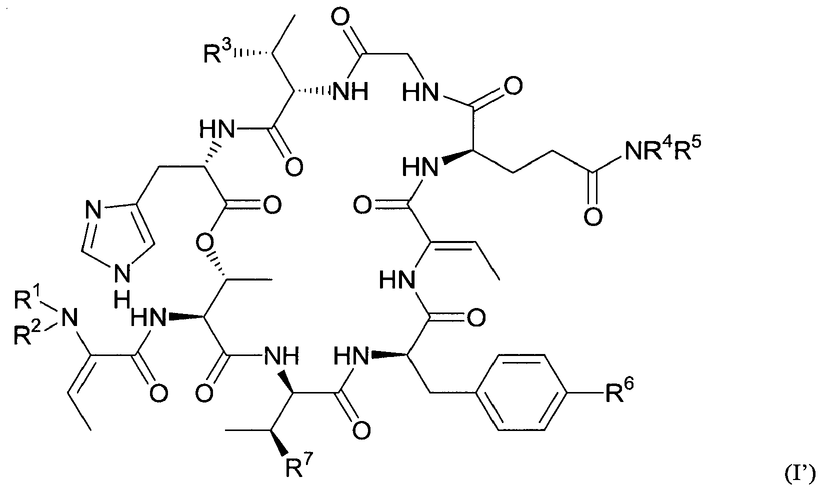 wherein R¹, R², R³, R⁴, R⁵, R⁶, R⁷ are defined as in claim 1.

3. The compound according to claim 1 or claim 2, or a pharmacologically acceptable salt thereof, wherein R¹ is a group represented by the general formula (II): 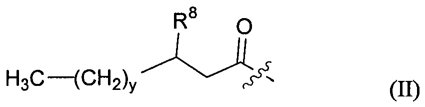 wherein
R⁸ can be a hydrogen atom, a halogen atom, a hydroxyl group, a cyano group, a nitro group, an amino, a mercapto group, an alkyl group having 1 to 6 carbon atoms in which one or more hydrogen atoms optionally have been replaced each independently of the others by a fluorine, chlorine, bromine or iodine atom; or by OH, =O, SH, =S, NH₂ =NH, CN, NO₂, or an alkoxy group, an alkoxy group having 1 to 6 carbon atoms, a SOCH₃ group, a SO₂CH₃ group, an acyl group having 2 to 6 carbon atoms, or a polyethylene glycol group of formula -A-[CH₂-CH₂-O]ₙ-R²⁰, wherein A is -0-, -C(=O)-, - OC(=O)-, or -OC(=O)- (CH₂)ₘ-O-; m is an integer from 1 to 20; n is an integer of from 2 to 100; and R²⁰ is a hydrogen atom, a methyl group, or an ethyl group; and
y is an integer from 1 to 20.

4. The compound according to any one of claims 1 to 3, or a pharmacologically acceptable salt thereof, wherein R¹ is a group represented by the general formula (II'): 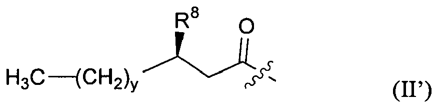 wherein R⁸ and y are defined as in claim 3.

5. The compound according to any one of claims 1 to 4, or a pharmacologically acceptable salt thereof, wherein R² is hydrogen atom or an alkyl group having 1 to 6 carbon atoms in which one or more hydrogen atoms optionally have been replaced each independently of the others by a fluorine, chlorine, bromine or iodine atom; or by OH, =O, SH, =S, NH₂, =NH, CN, NO₂, or an alkoxy group.

6. The compound according to any one of claims 1 to 5, or a pharmacologically acceptable salt thereof, wherein R³ is a halogen atom, a hydroxyl group, an amino group, or an alkyl group having 1 to 6 carbon atoms in which one or more hydrogen atoms optionally have been replaced each independently of the others by a fluorine, chlorine, bromine or iodine atom; or by OH, =O, SH, =S, NH₂, =NH, CN, NO₂, or an alkoxy group.

7. The compound according to any one of claims 1 to 6, or a pharmacologically acceptable salt thereof, wherein R⁴ and R⁵ each independently represents a hydrogen atom, or an alkyl group in which one or more hydrogen atoms optionally have been replaced each independently of the others by a fluorine, chlorine, bromine or iodine atom; or by OH, =O, SH, =S, NH₂, =NH, CN, NO₂, or an alkoxy group, wherein one carbon atom in said alkyl group may be replaced by an oxygen atom, a sulfur atom, C=O, NR¹³, CONR¹⁴, or NR¹⁵CO at any chemically allowable position; and R¹³, R¹⁴ , and R¹⁵ are defined as in claim 1.

8. The compound according to any one of claims 1 to 7, or a pharmacologically acceptable salt thereof, wherein R⁶ is a halogen atom, a hydroxyl group, an amino group, an optionally substituted alkyl group having 1 to 6 carbon atoms in which one or more hydrogen atoms optionally have been replaced each independently of the others by a fluorine, chlorine, bromine or iodine atom; or by OH, =O, SH, =S, NH₂, =NH, CN, NO₂, or an alkoxy group, an alkoxy group having 1 to 6 carbon atoms, a SOCH₃ group, a SO₂CH₃ group, or an acyl group having 2 to 6 carbon atoms.

9. The compound according to any one of claims 1 to 8, or a pharmacologically acceptable salt thereof, wherein R⁷ is a halogen atom, a hydroxyl group, an amino group, an optionally substituted alkyl group having 1 to 6 carbon atoms in which one or more hydrogen atoms optionally have been replaced each independently of the others by a fluorine, chlorine, bromine or iodine atom; or by OH, =O, SH, =S, NH₂, =NH, CN, NO₂, or an alkoxy group, an alkoxy group having 1 to 6 carbon atoms, a SOCH₃ group, a SO₂CH₃ group, an acyl group having 2 to 6 carbon atoms, or a polyethylene glycol group of formula -A-[CH₂-CH₂-O]ₙ-R²⁰, wherein A -C(=O)-, or -OC(=O)- (CH₂)ₘ-O-; m is an integer of from 1 to 6; n is an integer of from 2 to 10, and R²⁰ is a hydrogen atom, or a methyl group.

10. The compound according to any one of claims 1 to 9, or a pharmacologically acceptable salt thereof, wherein the compound is represented by formula (III):

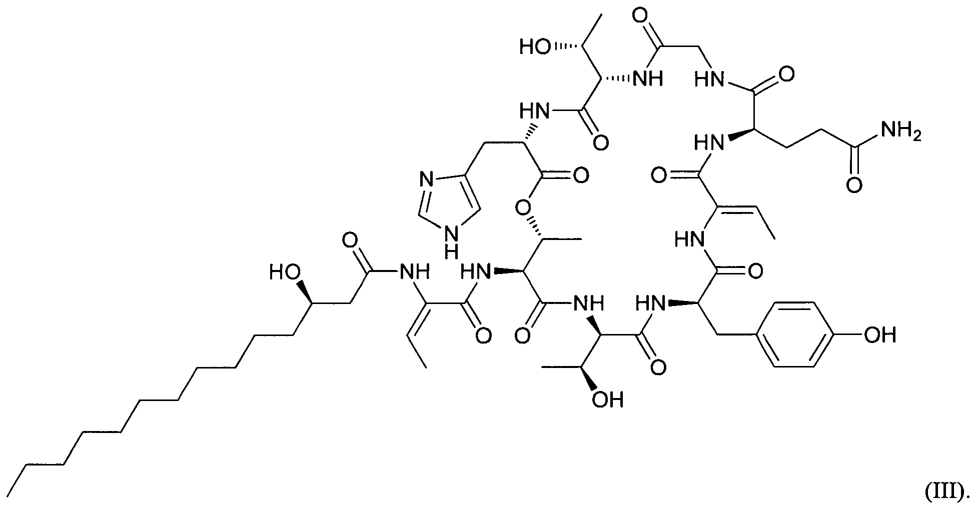

11. A pharmaceutical composition that comprises at least one compound according to any one of claims 1 to 10, or a pharmacologically acceptable salt thereof and, optionally, at least one carrier substance and/or at least one adjuvant.

12. A method for the preparation of a compound of formula (I), the method comprising the steps of:
(a) fermenting *Janthinobacterium agaricidamnosum* (DSM 9628); and
(b) separating and retaining the compound from the culture broth; wherein the compound is a compound according to claim 10.

13. The compound, or a pharmacologically acceptable salt thereof, or the pharmaceutical composition according to any one of claims 1 to 11 for use as a medicament.

14. The compound, or a pharmacologically acceptable salt thereof, or the pharmaceutical composition according to any one of claims 1 to 11 for use in the treatment or prevention of a fungal infection or cancer.

## Patentansprüche

1. Verbindung der allgemeinen Formel (I): 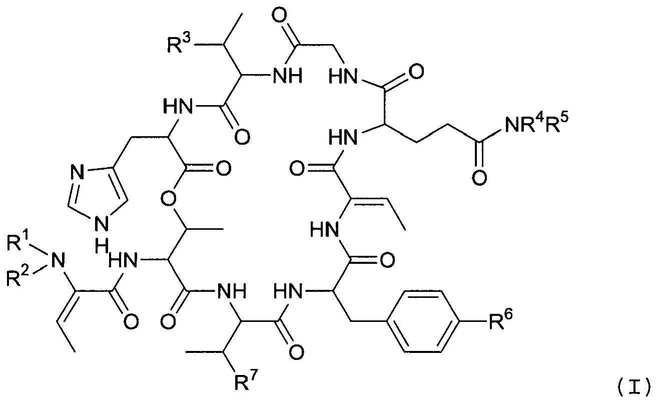 oder ein pharmakologisch akzeptables Salz derselben, wobei
R¹ und R² jeweils unabhängig voneinander ein Wasserstoffatom, eine Alkylgruppe, in der gegebenenfalls ein oder mehrere Wasserstoffatom(e) jeweils unabhängig von den anderen durch ein Fluor-, Chlor-, Brom- oder Jodatom; oder durch OH, =O, SH, =S, NH₂, =NH, CN, NO₂ oder eine Alkoxygruppe ersetzt worden sein kann / können; eine Alkenylgruppe, in der gegebenenfalls ein oder mehrere Wasserstoffatom(e) jeweils unabhängig von den anderen durch ein Fluor-, Chlor-, Brom- oder Jodatom; oder durch OH, =O, SH, =S, NH₂, =NH, CN, NO₂ oder eine Alkoxygruppe ersetzt worden sein kann / können; oder eine Alkinylgruppe, in der gegebenenfalls ein oder mehrere Wasserstoffatom(e) jeweils unabhängig von den anderen durch ein Fluor-, Chlor-, Brom-oder Jodatom; oder durch OH, =O, SH, =S, NH₂, =NH, CN, NO₂ oder eine Alkoxygruppe ersetzt worden sein kann / können, darstellt; und wobei ein Kohlenstoffatom in der Alkyl-, Alkenyl- oder Alkinylgruppe durch ein Sauerstoffatom, ein Schwefelatom, C=O, NR¹⁰, CONR¹¹ oder NR¹²CO an jeder chemisch erlaubten Position ersetzt sein kann;
R¹⁰, R¹¹, und R¹² jeweils unabhängig voneinander ein Wasserstoffatom oder eine Alkylgruppe mit 1 bis 6 Kohlenstoffatomen darstellt;
R³ ein Wasserstoffatom, ein Halogenatom, eine Hydroxylgruppe, eine Cyanogruppe, eine Nitrogruppe, eine Aminogruppe, eine Mercaptogruppe, eine Alkylgruppe mit 1 bis 6 Kohlenstoffatomen, in der gegebenenfalls ein oder mehrere Wasserstoffatom(e) jeweils unabhängig von den anderen durch ein Fluor-, Chlor-, Brom- oder Jodatom; oder durch OH, =O, SH, =S, NH₂, =NH, CN, NO₂ oder eine Alkoxygruppe ersetzt worden sein kann / können; eine Alkoxygruppe mit 1 bis 6 Kohlenstoffatomen, eine SOCH₃ -Gruppe, eine SO₂CH₃ -Gruppe, oder eine Acylgruppe mit 2 bis 6 Kohlenstoffatomen, oder eine Polyethylenglycolgruppe der Formel -A-[CH₂-CH₂-O]ₙ-R²⁰ ist, wobei A -O-, -C(=O)-, -OC(=O)-, oder -OC(=O)- (CH₂)ₘ-O- ist; m eine ganze Zahl von 1 bis 20 ist; n eine ganze Zahl von 2 bis 100 ist, und R²⁰ ein Wasserstoffatom, eine Methylgruppe, oder eine Ethylgruppe ist;
R⁴ und R⁵ jeweils unabhängig voneinander ein Wasserstoffatom, eine Alkylgruppe, in der gegebenenfalls ein oder mehrere Wasserstoffatom(e) jeweils unabhängig von den anderen durch ein Fluor-, Chlor-, Brom- oder Jodatom; oder durch OH, =O, SH, =S, NH₂, =NH, CN, NO₂ oder eine Alkoxygruppe ersetzt worden sein kann / können; eine Alkenylgruppe, in der gegebenenfalls ein oder mehrere Wasserstoffatom(e) jeweils unabhängig von den anderen durch ein Fluor-, Chlor-, Brom- oder Jodatom; oder durch OH, =O, SH, =S, NH₂, =NH, CN, NO₂ oder eine Alkoxygruppe ersetzt worden sein kann / können; oder eine Alkinylgruppe, in der gegebenenfalls ein oder mehrere Wasserstoffatom(e) jeweils unabhängig von den anderen durch ein Fluor-, Chlor-, Brom- oder Jodatom; oder durch OH, =O, SH, =S, NH₂, =NH, CN, NO₂ oder eine Alkoxygruppe ersetzt worden sein kann / können, darstellt; und wobei ein Kohlenstoffatom in der Alkyl-, Alkenyl- oder Alkinylgruppe durch ein Sauerstoffatom, ein Schwefelatom, C=O, NR¹⁰, CONR¹¹ oder NR¹²CO an jeder chemisch erlaubten Position ersetzt sein kann;
R¹³, R¹⁴, und R¹⁵ jeweils unabhängig voneinander ein Wasserstoffatom oder eine Alkylgruppe mit 1 bis 6 Kohlenstoffatomen darstellt;
R⁶ ein Wasserstoffatom, ein Halogenatom, eine Hydroxylgruppe, eine Cyanogruppe, eine Nitrogruppe, eine Aminogruppe, eine Mercaptogruppe, eine Alkylgruppe mit 1 bis 6 Kohlenstoffatomen, in der gegebenenfalls ein oder mehrere Wasserstoffatom(e) jeweils unabhängig von den anderen durch ein Fluor-, Chlor-, Brom- oder Jodatom; oder durch OH, =O, SH, =S, NH₂, =NH, CN, NO₂ oder eine Alkoxygruppe ersetzt worden sein kann / können; eine Alkoxygruppe mit 1 bis 6 Kohlenstoffatomen, eine SOCH₃ -Gruppe, eine SO₂CH₃ -Gruppe, oder eine Acylgruppe mit 2 bis 6 Kohlenstoffatomen, oder eine Polyethylenglycolgruppe der Formel -A- [CH₂-CH₂-O]ₙ-R²⁰ ist, wobei A -O-, -C(=O) -, -OC(=O)-, oder -OC(=O)- (CH₂)ₘ-O- ist; m eine ganze Zahl von 1 bis 20 ist; n eine ganze Zahl von 2 bis 100 ist, und R²⁰ ein Wasserstoffatom, eine Methylgruppe, oder eine Ethylgruppe ist; und
R⁷ ein Wasserstoffatom, ein Halogenatom, eine Hydroxylgruppe, eine Cyanogruppe, eine Nitrogruppe, eine Aminogruppe, eine Mercaptogruppe, eine Alkylgruppe mit 1 bis 6 Kohlenstoffatomen, in der gegebenenfalls ein oder mehrere Wasserstoffatom(e) jeweils unabhängig von den anderen durch ein Fluor-, Chlor-, Brom- oder Jodatom; oder durch OH, =O, SH, =S, NH₂, =NH, CN, NO₂ oder eine Alkoxygruppe ersetzt worden sein kann / können; eine Alkoxygruppe mit 1 bis 6 Kohlenstoffatomen, eine SOCH₃ -Gruppe, eine SO₂CH₃ -Gruppe, oder eine Acylgruppe mit 2 bis 6 Kohlenstoffatomen, oder eine Polyethylenglycolgruppe der Formel -A-[CH₂-CH₂-O]ₙ-R²⁰ ist, wobei A -O-, -C(=O)-, -OC(=O)-, oder -OC(=O)- (CH₂)ₘ-O- ist; m eine ganze Zahl von 1 bis 20 ist; n eine ganze Zahl von 2 bis 100 ist, und R²⁰ ein Wasserstoffatom, eine Methylgruppe, oder eine Ethylgruppe ist.

2. Die Verbindung nach Anspruch 1 oder ein pharmakologisch akzeptables Salz derselben, wobei die Verbindung durch die allgemeine Formel (I') dargestellt ist: 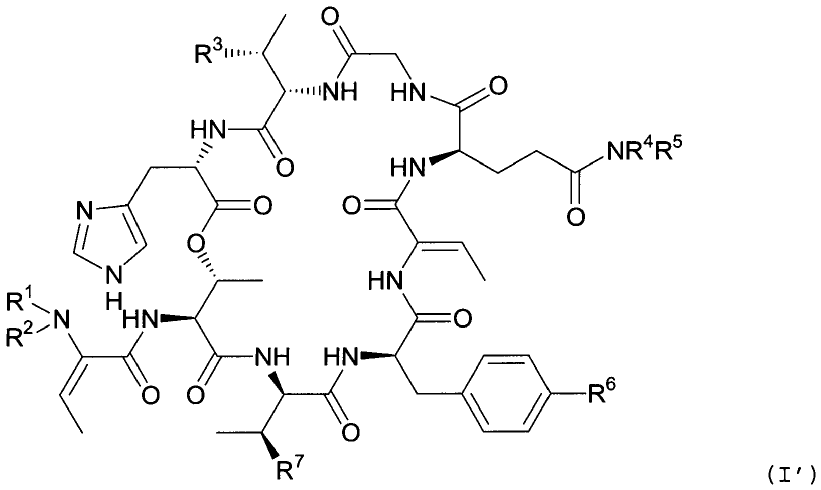 wobei R¹, R², R³, R⁴, R⁵, R⁶, R⁷ wie in Anspruch 1 definiert sind.

3. Die Verbindung nach Anspruch 1 oder Anspruch 2, oder ein pharmakologisch akzeptables Salz derselben, wobei R¹ eine Gruppe ist, die durch die allgemeine Formel (II) dargestellt ist: 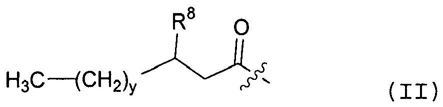 wobei
R⁸ ein Wasserstoffatom, ein Halogenatom, eine Hydroxylgruppe, eine Cyanogruppe, eine Nitrogruppe, eine Aminogruppe, eine Mercaptogruppe, eine Alkylgruppe mit 1 bis 6 Kohlenstoffatomen, in der gegebenenfalls ein oder mehrere Wasserstoffatom(e) jeweils unabhängig von den anderen durch ein Fluor-, Chlor-, Brom- oder Jodatom; oder durch OH, =O, SH, =S, NH₂, =NH, CN, NO₂ oder eine Alkoxygruppe ersetzt worden sein kann / können; eine Alkoxygruppe mit 1 bis 6 Kohlenstoffatomen, eine SOCH₃ -Gruppe, eine SO₂CH₃ -Gruppe, oder eine Acylgruppe mit 2 bis 6 Kohlenstoffatomen, oder eine Polyethylenglycolgruppe der Formel -A-[CH₂-CH₂-O]ₙ-R²⁰ sein kann, wobei A -O-, -C(=O)-, -OC(=O)-, oder -OC(=O)- (CH₂)ₘ-O-ist; m eine ganze Zahl von 1 bis 20 ist; n eine ganze Zahl von 2 bis 100 ist, und R²⁰ ein Wasserstoffatom, eine Methylgruppe, oder eine Ethylgruppe ist; und
y eine ganze Zahl von 1 bis 20 ist.

4. Die Verbindung nach einem der Ansprüche 1 bis 3, oder ein pharmakologisch akzeptables Salz derselben, wobei R¹ eine Gruppe ist, die durch die allgemeine Formel (II') dargestellt ist: 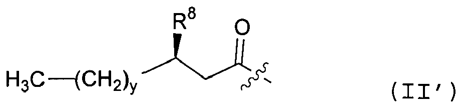 wobei R⁸ und y wie in Anspruch 3 definiert sind.

5. Die Verbindung nach einem der Ansprüche 1 bis 4, oder ein pharmakologisch akzeptables Salz derselben, wobei R² ein Wasserstoffatom oder eine Alkylgruppe mit 1 bis 6 Kohlenstoffatomen, in der gegebenenfalls ein oder mehrere Wasserstoffatom(e) jeweils unabhängig von den anderen durch ein Fluor-, Chlor-, Brom- oder Jodatom; oder durch OH, =O, SH, =S, NH₂, =NH, CN, NO₂ oder eine Alkoxygruppe ersetzt worden sein kann / können, ist.

6. Die Verbindung nach einem der Ansprüche 1 bis 5, oder ein pharmakologisch akzeptables Salz derselben, wobei R³ ein Halogenatom, eine Hydroxylgruppe, eine Aminogruppe, oder eine Alkylgruppe mit 1 bis 6 Kohlenstoffatomen, in der gegebenenfalls ein oder mehrere Wasserstoffatom(e) jeweils unabhängig von den anderen durch ein Fluor-, Chlor-, Brom-oder Jodatom; oder durch OH, =O, SH, =S, NH₂, =NH, CN, NO₂ oder eine Alkoxygruppe ersetzt worden sein kann / können, ist.

7. Die Verbindung nach einem der Ansprüche 1 bis 6, oder ein pharmakologisch akzeptables Salz derselben, wobei R⁴ und R⁵ jeweils unabhängig voneinander ein Wasserstoffatom, oder eine Alkylgruppe, in der gegebenenfalls ein oder mehrere Wasserstoffatom(e) jeweils unabhängig von den anderen durch ein Fluor-, Chlor-, Brom- oder Jodatom; oder durch OH, =O, SH, =S, NH₂, =NH, CN, NO₂ oder eine Alkoxygruppe ersetzt worden sein kann / können, darstellt; wobei ein Kohlenstoffatom in der Alkylgruppe durch ein Sauerstoffatom, ein Schwefelatom, C=O, NR¹³, CONR¹⁴ oder NR¹⁵CO an jeder chemisch erlaubten Position ersetzt sein kann; und R¹³, R¹⁴, und R¹⁵ wie in Anspruch 1 definiert sind.

8. Die Verbindung nach einem der Ansprüche 1 bis 7, oder ein pharmakologisch akzeptables Salz derselben, wobei R⁶ ein Halogenatom, eine Hydroxylgruppe, eine Aminogruppe, eine Alkylgruppe mit 1 bis 6 Kohlenstoffatomen, in der gegebenenfalls ein oder mehrere Wasserstoffatom(e) jeweils unabhängig von den anderen durch ein Fluor-, Chlor-, Brom-oder Jodatom; oder durch OH, =O, SH, =S, NH₂, =NH, CN, NO₂ oder eine Alkoxygruppe ersetzt worden sein kann / können; eine Alkoxygruppe mit 1 bis 6 Kohlenstoffatomen, eine SOCH₃ -Gruppe, eine SO₂CH₃ -Gruppe, oder eine Acylgruppe mit 2 bis 6 Kohlenstoffatomen ist.

9. Die Verbindung nach einem der Ansprüche 1 bis 8, oder ein pharmakologisch akzeptables Salz derselben, wobei R⁷ ein Halogenatom, eine Hydroxylgruppe, eine Aminogruppe, eine gegebenenfalls substituierte Alkylgruppe mit 1 bis 6 Kohlenstoffatomen, in der gegebenenfalls ein oder mehrere Wasserstoffatom(e) jeweils unabhängig von den anderen durch ein Fluor-, Chlor-, Brom- oder Jodatom; oder durch OH, =O, SH, =S, NH₂, =NH, CN, NO₂ oder eine Alkoxygruppe ersetzt worden sein kann / können; eine Alkoxygruppe mit 1 bis 6 Kohlenstoffatomen, eine SOCH₃ -Gruppe, eine SOCH₃ -Gruppe, eine Acylgruppe mit 2 bis 6 Kohlenstoffatomen, oder eine Polyethylenglycolgruppe der Formel -A-[CH₂-CH₂-O]ₙ-R²⁰ ist, wobei A -C(=O)-, oder -OC(=O)- (CH₂)ₘ-O- ist; m eine ganze Zahl von 1 bis 6 ist; n eine ganze Zahl von 2 bis 10 ist, und R²⁰ ein Wasserstoffatom oder eine Methylgruppe ist.

10. Die Verbindung nach einem der Ansprüche 1 bis 9, oder ein pharmakologisch akzeptables Salz derselben, wobei die Verbindung durch die allgemeine Formel (III) dargestellt ist:

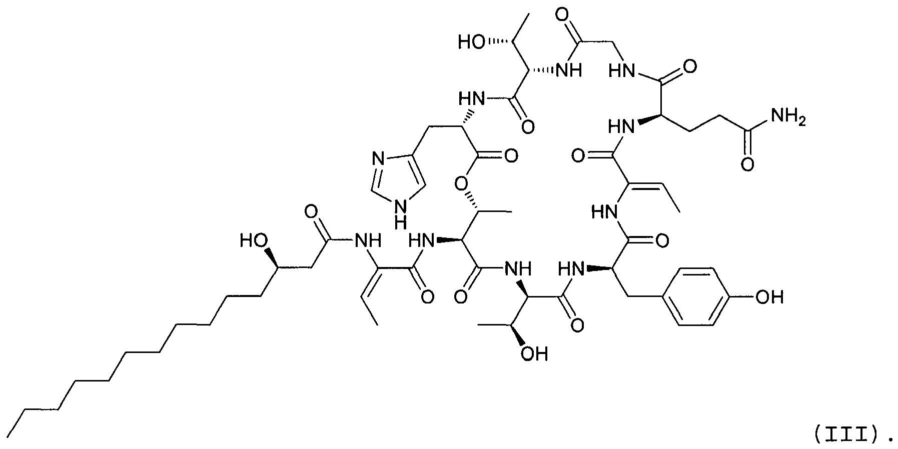

11. Pharmazeutische Zusammensetzung, die mindestens eine Verbindung gemäß einem der Ansprüche 1 bis 10, oder ein pharmakologisch akzeptables Salz derselben, und gegebenenfalls mindestens eine Trägersubstanz und / oder mindestens ein Adjuvans umfasst.

12. Verfahren zur Herstellung einer Verbindung gemäß der Formel (I), wobei das Verfahren die Schritte umfasst:
(a) *Janthinobacterium agaricidamnosum* (DSM 9628) fermentieren; und
(b) Abtrennen und Gewinnen der Verbindung aus dem Kulturmedium; wobei die Verbindung eine Verbindung gemäß Anspruch 10 ist.

13. Die Verbindung, oder ein pharmakologisch akzeptables Salz derselben, oder die pharmazeutische Zusammensetzung nach einem der Ansprüche 1 bis 11 zur Verwendung als Medikament.

14. Die Verbindung, oder ein pharmakologisch akzeptables Salz derselben, oder die pharmazeutische Zusammensetzung nach einem der Ansprüche 1 bis 11 zur Verwendung bei der Behandlung oder Prophylaxe einer Pilzinfektion oder von Krebs.

## Revendications

1. Composé de la formule générale (I) : 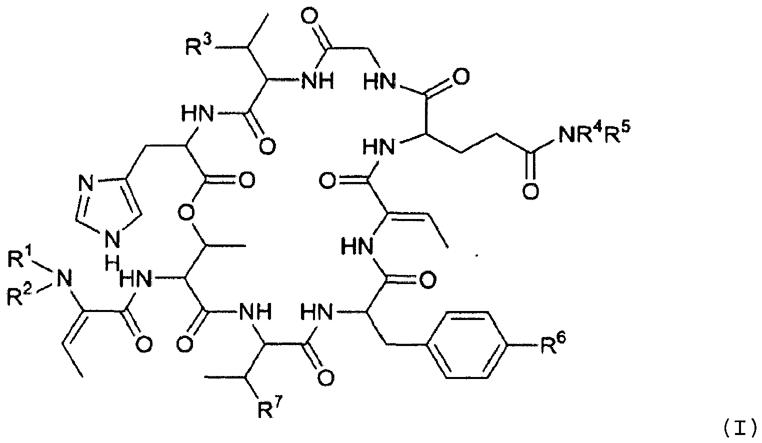 ou un sel pharmacologiquement acceptable de celui-ci, où
R¹ et R² représentent chacun indépendamment un atome d'hydrogène, un groupe alkyle dans lequel un ou plusieurs atome(s) d'hydrogène a/ont été éventuellement remplacé(s) chacun indépendamment des autres par un atome de fluor, de chlore, de brome ou d'iode ; ou par OH, =O, SH, =S, NH₂, =NH, CN, NO₂, ou un groupe alcoxy, un groupe alcényle dans lequel un ou plusieurs atome(s) d'hydrogène a/ont été éventuellement remplacé(s) chacun indépendamment des autres par un atome de fluor, de chlore, de brome ou d'iode ; ou par OH, =O, SH, =S, NH₂, =NH, CN, NO₂, ou un groupe alcoxy, ou un groupe alcynyle dans lequel un ou plusieurs atome(s) d'hydrogène a/ont été éventuellement remplacé(s) chacun indépendamment des autres par un atome de fluor, de chlore, de brome ou d'iode ; ou par OH, =O, SH, =S, NH₂, =NH, CN, NO₂, ou un groupe alcoxy, et où un atome de carbone dans ledit groupe alkyle, alcényle ou alcynyle peut être remplacé par un atome d'oxygène, un atome de soufre, C=O, NR¹⁰, CONR¹¹, ou NR¹²CO en toute position chimiquement permise;
R¹⁰, R¹¹, et R¹² représentent chacun indépendamment un atome d'hydrogène ou un groupe alkyle ayant 1 à 6 atomes de carbone;
R³ est un atome d'hydrogène, un atome d'halogène, un groupe hydroxyle, un groupe cyano, un groupe nitro, un groupe amino, un groupe mercapto, un groupe alkyle ayant 1 à 6 atomes de carbone dans lequel un ou plusieurs atome(s) d'hydrogène a/ont été éventuellement remplacé(s) chacun indépendamment des autres par un atome de fluor, de chlore, de brome ou d'iode ; ou par OH, =O, SH, =S, NH₂, =NH, CN, NO₂, ou un groupe alcoxy, un groupe alcoxy ayant 1 à 6 atomes de carbone, un groupe SOCH₃, un groupe SO₂CH₃, ou un groupe acyle ayant 2 à 6 atomes de carbone, ou un groupe polyéthylène glycol de formule -A-[CH₂-CH₂-O]ₙ-R²⁰, dans lequel A est -O-, -C(=O)-, -OC(=O)-, ou - OC(=O)-(CH₂)ₘ-O- ; m est un nombre entier compris entre 1 et 20 ; n est un nombre entier compris entre 2 et 100, et R²⁰ est un atome d'hydrogène, un groupe méthyle ou un groupe éthyle;
R⁴ et R⁵ représentent chacun indépendamment un atome d'hydrogène, un groupe alkyle dans lequel un ou plusieurs atome(s) d'hydrogène a/ont été éventuellement remplacé(s) chacun indépendamment des autres par un atome de fluor, de chlore, de brome ou d'iode ; ou par OH, =O, SH, =S, NH₂, =NH, CN, NO₂, ou un groupe alcoxy, un groupe alcényle dans lequel un ou plusieurs atome(s) d'hydrogène a/ont été éventuellement remplacé(s) chacun indépendamment des autres par un atome de fluor, de chlore, de brome ou d'iode ; ou par OH, =O, SH, =S, NH₂, =NH, CN, NO₂, ou un groupe alcoxy, ou un groupe alcynyle dans lequel un ou plusieurs atome(s) d'hydrogène a/ont été éventuellement remplacé(s) chacun indépendamment des autres par un atome de fluor, de chlore, de brome ou d'iode ; ou par OH, =O, SH, =S, NH₂, =NH, CN, NO₂, ou un groupe alcoxy, où un atome de carbone dans ledit groupe alkyle, alcényle ou alcynyle peut être remplacé par un atome d'oxygène, un atome de soufre, C=O, NR¹³, CONR¹⁴, ou NR¹⁵CO en toute position chimiquement permise;
R¹³, R¹⁴, et R¹⁵ représentent chacun indépendamment un atome d'hydrogène ou un groupe alkyle ayant 1 à 6 atomes de carbone;
R⁶ est un atome d'hydrogène, un atome d'halogène, un groupe hydroxyle, un groupe cyano, un groupe nitro, un groupe amino, un groupe mercapto, un groupe alkyle ayant 1 à 6 atomes de carbone dans lequel un ou plusieurs atome(s) d'hydrogène a/ont été éventuellement remplacé(s) chacun indépendamment des autres par un atome de fluor, de chlore, de brome ou d'iode ; ou par OH, =O, SH, =S, NH₂, =NH, CN, NO₂, ou un groupe alcoxy, un groupe alcoxy ayant 1 à 6 atomes de carbone, un groupe SOCH₃, un groupe SO₂CH₃, un groupe acyle ayant 2 à 6 atomes de carbone, ou un groupe polyéthylène glycol de formule -A-[CH₂-CH₂-O]ₙ-R²⁰, dans lequel A est -O-, -C(=O)-, -OC(=O)-, ou - OC(=O)-(CH₂)ₘ-O- ; m est un nombre entier compris entre 1 et 20 ; n est un nombre entier compris entre 2 et 100, et R²⁰ est un atome d'hydrogène, un groupe méthyle ou un groupe éthyle ; et
R⁷ est un atome d'hydrogène, un atome d'halogène, un groupe hydroxyle, un groupe cyano, un groupe nitro, un groupe amino, un groupe mercapto, un groupe alkyle ayant 1 à 6 atomes de carbone dans lequel un ou plusieurs atome(s) d'hydrogène a/ont été éventuellement remplacé(s) chacun indépendamment des autres par un atome de fluor, de chlore, de brome ou d'iode ; ou par OH, =O, SH, =S, NH₂, =NH, CN, NO₂, ou un groupe alcoxy, un groupe alcoxy ayant 1 à 6 atomes de carbone, un groupe SOCH₃, un groupe SO₂CH₃, ou un groupe acyle ayant 2 à 6 atomes de carbone, ou un groupe polyéthylène glycol de formule -A-[CH₂-CH₂-O]ₙ-R²⁰, où A est --O-, -C(=O)-, -OC(=O) -, ou -OC(=O) - (CH₂)ₘ-O- ; m est un nombre entier compris entre 1 et 20 ; n est un nombre entier compris entre 2 et 100, et R²⁰ est un atome d'hydrogène, un groupe méthyle ou un groupe éthyle.

2. Composé selon la revendication 1 ou un sel pharmacologiquement acceptable de celui-ci, dans lequel le composé est représenté par la formule générale (I') : 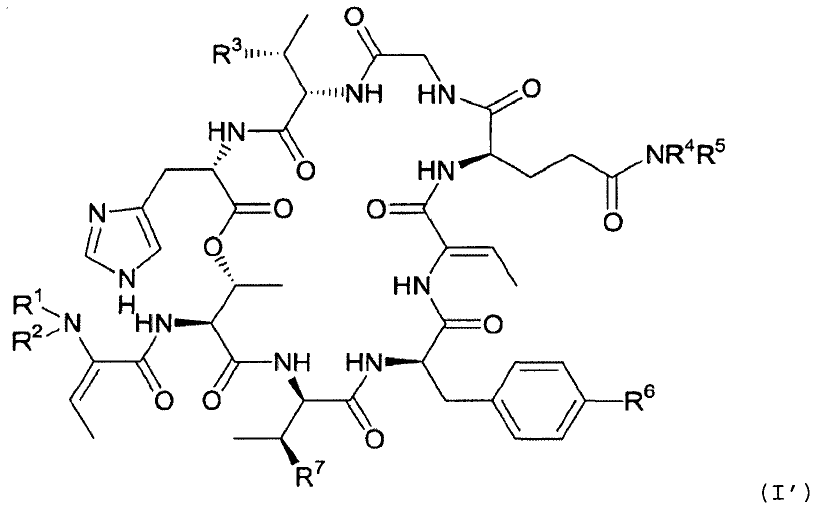 où R¹, R², R³, R⁴, R⁵, R⁶, R⁷ sont tels que définis dans la revendication 1.

3. Composé selon la revendication 1 ou 2, ou un sel pharmacologiquement acceptable de celui-ci, dans lequel R¹ est un groupe représenté par la formule générale (II) : 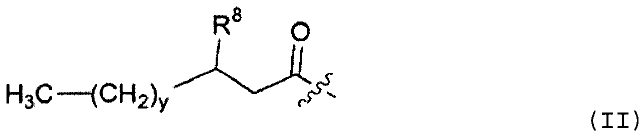 où
R⁸ peut être un atome d'hydrogène, un atome d'halogène, un groupe hydroxyle, un groupe cyano, un groupe nitro, un groupe amino, un groupe mercapto, un groupe alkyle ayant 1 à 6 atomes de carbone dans lequel un ou plusieurs atome(s) d'hydrogène a/ont été éventuellement remplacé(s) chacun indépendamment des autres par un atome de fluor, de chlore, de brome ou d'iode ; ou par OH, =O, SH, =S, NH₂, =NH, CN, NO₂, ou un groupe alcoxy, un groupe alcoxy ayant 1 à 6 atomes de carbone, un groupe SOCH₃, un groupe SO₂CH₃, ou un groupe acyle ayant 2 à 6 atomes de carbone, ou un groupe polyéthylène glycol de formule -A-[CH₂-CH₂-O]ₙ-R²⁰, où A est -O-, -C(=O)-, -OC (=O) -, ou -OC (=O) - (CH₂)ₘ-O- ; m est un nombre entier compris entre 1 et 20 ; n est un nombre entier compris entre 2 et 100, et R²⁰ est un atome d'hydrogène, un groupe méthyle ou un groupe éthyle ; et
y est un nombre entier compris entre 1 et 20.

4. Composé selon l'une quelconque des revendications 1 à 3, ou un sel pharmacologiquement acceptable de celui-ci, dans lequel R¹ est un groupe représenté par la formule générale (II') : 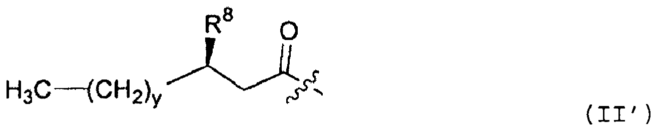 où R⁸ et Y sont tels que définis dans la revendication 3.

5. Composé selon l'une quelconque des revendications 1 à 4, ou un sel pharmacologiquement acceptable de celui-ci, dans lequel R² est un atome d'hydrogène ou un groupe alkyle ayant 1 à 6 atomes de carbone dans lequel un ou plusieurs atome(s) d'hydrogène a/ont été éventuellement remplacé(s) chacun indépendamment des autres par un atome de fluor, de chlore, de brome ou d'iode ; ou par OH, =O, SH, =S, NH₂, =NH, CN, NO₂, ou un groupe alcoxy.

6. Composé selon l'une quelconque des revendications 1 à 5, ou un sel pharmacologiquement acceptable de celui-ci, dans lequel R³ est un atome d'halogène, un groupe hydroxyle, un groupe amino, ou un groupe alkyle ayant 1 à 6 atomes de carbone dans lequel un ou plusieurs atome(s) d'hydrogène a/ont été éventuellement remplacé(s) chacun indépendamment des autres par un atome de fluor, de chlore, de brome ou d'iode ; ou par OH, =O, SH, =S, NH₂, =NH, CN, NO₂, ou un groupe alcoxy.

7. Composé selon l'une quelconque des revendications 1 à 6, ou un sel pharmacologiquement acceptable de celui-ci, dans lequel R⁴ et R⁵ représentent chacun indépendamment un atome d'hydrogène ou un groupe alkyle dans lequel un ou plusieurs atome(s) d'hydrogène a/ont été éventuellement remplacé(s) chacun indépendamment des autres par un atome de fluor, de chlore, de brome ou d'iode ; ou par OH, =O, SH, =S, NH₂, =NH, CN, NO₂, ou un groupe alcoxy, où un atome de carbone dans ledit groupe alkyle peut être remplacé par un atome d'oxygène, un atome de soufre, C=O, NR¹³, CONR¹⁴, ou NR¹⁵CO en toute position chimiquement permise ; et R¹³, R¹⁴, et R¹⁵ sont tels que définis dans la revendication 1.

8. Composé selon l'une quelconque des revendications 1 à 7, ou un sel pharmacologiquement acceptable de celui-ci, dans lequel R⁶ est un atome d'halogène, un groupe hydroxyle, un groupe amino, un groupe alkyle ayant 1 à 6 atomes de carbone dans lequel un ou plusieurs atome(s) d'hydrogène a/ont été éventuellement remplacé(s) chacun indépendamment des autres par un atome de fluor, de chlore, de brome ou d'iode ; ou par OH, =O, SH, =S, NH₂, =NH, CN, NO₂, ou un groupe alcoxy, un groupe alcoxy ayant 1 à 6 atomes de carbone, un groupe SOCH₃, un groupe SO₂CH₃, ou un groupe acyle ayant 2 à 6 atomes de carbone.

9. Composé selon l'une quelconque des revendications 1 à 8, ou un sel pharmacologiquement acceptable de celui-ci, dans lequel R⁷ est un atome d'halogène, un groupe hydroxyle, un groupe amino, un groupe alkyle ayant 1 à 6 atomes de carbone dans lequel un ou plusieurs atome(s) d'hydrogène a/ont été éventuellement remplacé(s) chacun indépendamment des autres par un atome de fluor, de chlore, de brome ou d'iode ; ou par OH, =O, SH, =S, NH₂, =N_{H}, CN, NO₂, ou un groupe alcoxy, un groupe alcoxy ayant 1 à 6 atomes de carbone, un groupe SOCH₃, un groupe SO₂CH₃, ou un groupe acyle ayant 2 à 6 atomes de carbone, ou un groupe polyéthylène glycol de formule -A- [CH₂-CH₂-O]ₙ-R²⁰, où A est -C(=O) -, ou -OC(=O)-(CH₂)ₘ-O- ; m est un nombre entier compris entre 1 et 6 ; n est un nombre entier compris entre 2 et 10, et R²⁰ est un atome d'hydrogène ou un groupe méthyle.

10. Composé selon l'une quelconque des revendications 1 à 9, ou un sel pharmacologiquement acceptable de celui-ci, dans lequel le composé est représenté par la formule (III) :

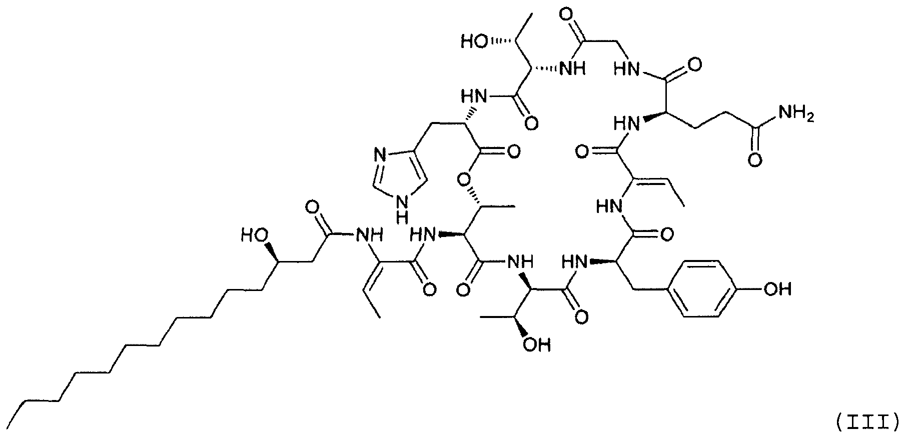

11. Composition pharmaceutique qui comprend au moins un composé selon l'une quelconque des revendications 1 à 10, ou un sel pharmacologiquement acceptable de celui-ci et, éventuellement, au moins une substance porteuse et/ou au moins un adjuvant.

12. Procédé pour la préparation d'un composé de formule (I), le procédé comprenant les étapes suivantes :
(a) la fermentation de *Janthinobacterium agaricidamnosum* (DSM 9628) ; et
(b) la séparation et la rétention du composé provenant du bouillon de culture ; où le composé est un composé selon la revendication 10.

13. Composé, ou un sel pharmacologiquement acceptable de celui-ci, ou la composition pharmaceutique selon l'une quelconque des revendications 1 à 11 pour une utilisation comme médicament.

14. Composé, ou un sel pharmacologiquement acceptable de celui-ci, ou la composition pharmaceutique selon l'une quelconque des revendications 1 à 11 pour une utilisation dans le traitement ou la prévention d'une infection fongique ou d'un cancer.
